# EUROPEAN PATENT SPECIFICATION

(11) **EP 3 897 546 B1**
(45) Date of publication and mention of the grant of the patent: **12.11.2025**
(21) Application number: 19827720.4
(22) Date of filing: 19.12.2019
(51) Int. Cl.: A61K 8/34, A61K 8/36, A61K 8/44, A61Q 5/06, A61Q 5/12, A61K 8/04, A61K 8/19

(54) **AEROSOL DEVICE CONTAINING A COSMETIC COMPOSITION COMPRISING AN ANIONIC SURFACTANT, A FATTY ALCOHOL AND A COMPRESSED GAS**
AEROSOLVORRICHTUNG MIT EINER KOSMETISCHEN ZUSAMMENSETZUNG MIT EINEM ANIONISCHEN TENSID, EINEM FETTALKOHOL UND EINEM KOMPRIMIERTEN GAS
DISPOSITIF AÉROSOL CONTENANT UNE COMPOSITION COSMÉTIQUE COMPRENANT UN TENSIOACTIF ANIONIQUE, UN ALCOOL GRAS ET UN GAZ COMPRIMÉ

(30) Priority: 21.12.2018 FR 1873796
(43) Date of publication of application: 27.10.2021
(73) Proprietor: L'OREAL, 75008 Paris (FR)
(72) Inventor: AUBERT, Lionel, 93400 SAINT OUEN (FR); BEAU, Nathalie, 93400 SAINT-OUEN (FR)
(74) Representative: Casalonga
(86) International application number: PCT/EP2019/086335
(87) International publication number: WO 2020/127755

(56) References cited:
- EP-A1- 2 130 530
- EP-A1- 2 213 333
- WO-A1-2016/172487
- CH-A2- 706 323
- US-A1- 2005 112 084
- DATABASE GNPD [online] MINTEL; 10 October 2018 (2018-10-10), ANONYMOUS: "Nourishing Cream Foam", XP055609274, retrieved from www.gnpd.com Database accession no. 6043681

## Description

The invention relates to an aerosol device containing a cosmetic composition comprising at least one anionic surfactant, at least one fatty alcohol, in a particular weight ratio, and a compressed gas as propellant.

The invention also relates to a cosmetic process for treating keratin fibres, in particular human keratin fibres such as the hair, comprising a step of applying to said keratin fibres said cosmetic composition sprayed from said aerosol device

The invention also relates to the use of said cosmetic composition sprayed from said aerosol device for conditioning and/or shaping keratin fibres, in particular human keratin fibres such as the hair.

Hairstyling products are normally used to construct and structure the hairstyle and to give it hold. They are usually in the form of lotions, gels, foams, creams or sprays. These compositions generally comprise one or more film-forming polymers or "fixing polymers". These polymers allow the formation of a coating film on the hair, thus providing hairstyle hold.

Hairstyling aid products or hairstyle hold products not comprising any fixing polymer also exist.

These compositions may be in the form of hair gels, sprays or foams which are generally applied to wet hair, which is shaped before performing blow-drying or drying.

The hairstyling foams dispensed from aerosol devices that are present on the market are generally very airy and expanded, with a light texture. The foam thus obtained is not sufficiently firm and lacks creaminess, which makes it difficult to apply. These foams also have a matt appearance which consumers find rather unpleasant.

Now, consumers are in search of hairstyling products, in particular in foam form, which are easy to apply and pleasant to use, while at the same time affording good sensory and styling properties to the keratin fibres.

Thus, there is a real need to provide a composition for conditioning and/or shaping keratin materials, in particular human keratin materials such as the hair, which does not have the drawbacks mentioned above, i.e. a composition with good foam properties and good formulation properties, notably in terms of the look and the feel, while at the same time being easy to apply and affording good styling properties, notably in terms of curl discipline, curl relaxing, curl definition, and anti-frizz effect.

It has been discovered, surprisingly, that an aerosol device containing a cosmetic composition comprising a combination of one or more anionic surfactants and one or more fatty alcohols, in a particular weight ratio, with one or more propellants, makes it possible to achieve the objectives presented above.

Specifically, dispensing this composition using an aerosol device makes it possible to form a foam which has a creamy and consistent texture with a glossy appearance.

In addition, this foam transforms into a more or less thick cream on application, ranging as far as a milk consistency for the most fluid. The cream thus obtained is easy to apply to keratin fibres.

The cosmetic composition of the invention also imparts satisfactory styling properties, notably in terms of curl discipline, curl relaxing, curl definition, and anti-frizz effect.

One subject of the present invention is thus an aerosol device containing a cosmetic composition comprising:
- one or more anionic surfactants,
- one or more fatty alcohols, and
- one or more propellants chosen from compressed gases,
the weight ratio between the total content of the fatty alcohol(s) and the total content of the anionic surfactant(s) ranging from 1.5 to 10.

A subject of the invention is also a cosmetic process for treating keratin fibres, in particular human keratin fibres such as the hair, comprising a step of applying to said keratin fibres a cosmetic composition sprayed from an aerosol device as defined previously.

A subject of the invention is also the use of a cosmetic composition sprayed from an aerosol device as defined previously, for conditioning and/or shaping keratin fibres.

Other subjects, characteristics, aspects and advantages of the invention will emerge even more clearly on reading the description and the examples that follow.

In the text hereinbelow, and unless otherwise indicated, the limits of a range of values are included within that range, notably in the expressions "between" and "ranging from ... to ...".

The expression "at least one" used in the present description is equivalent to the expression "one or more".

The composition according to the invention may comprise water. The composition is preferably aqueous. The water content preferably ranges from 10% to 99%, preferentially from 30% to 98%, better still from 50% to 97% by weight and even better still from 80% to 96% by weight relative to the total weight of the composition. Mention is made of the prior art document CH706323A1 which concerns cosmetic compositions contained in an aerosol container.

### Anionic surfactants

The composition contained in the aerosol device according to the present invention comprises one or more anionic surfactants.

The term "anionic surfactant" means a surfactant including, as ionic or ionizable groups, only anionic groups.

In the present description, a species is termed "anionic" when it bears at least one permanent negative charge or when it can be ionized into a negatively charged species, under the conditions of use of the composition of the invention (for example the medium or the pH) and not comprising any cationic charge.

The anionic surfactants may be chosen from sulfate, sulfonate and/or carboxylic (or carboxylate) surfactants. Needless to say, a mixture of these surfactants may be used.

It is understood in the present description that:
- the carboxylate anionic surfactants comprise at least one carboxylic or carboxylate function (-COOH or -COO⁻) and may optionally also comprise one or more sulfate and/or sulfonate functions;
- the sulfonate anionic surfactants comprise at least one sulfonate function (-SO₃H or -SO₃⁻) and may optionally also comprise one or more sulfate functions, but do not comprise any carboxylate functions; and
- the sulfate anionic surfactants comprise at least one sulfate function but do not comprise any carboxylate or sulfonate functions.

The carboxylate anionic surfactants that may be used thus include at least one carboxylic or carboxylate function (-COOH or -COO⁻).

They may be chosen from the following compounds: fatty acids, acylglycinates, acyllactylates, acylsarcosinates, acylglutamates; alkyl-D-galactosiduronic acids, alkyl ether carboxylic acids, alkyl(C₆-C₃₀ aryl) ether carboxylic acids, alkylamido ether carboxylic acids; and also the salts of these compounds; and mixtures thereof;
the alkyl and/or acyl groups of these compounds including from 6 to 30 carbon atoms, notably from 12 to 28, even better still from 14 to 24 or even from 16 to 22 carbon atoms; the aryl group preferably denoting a phenyl or benzyl group;
these compounds possibly being polyoxyalkylenated, notably polyoxyethylenated, and then preferably including from 1 to 50 ethylene oxide units and better still from 2 to 10 ethylene oxide units.

Use may also be made of C₆-C₂₄ alkyl monoesters of polyglycoside-polycarboxylic acids such as C₆-C₂₄ alkyl polyglycoside-citrates, C₆-C₂₄ alkyl polyglycoside-tartrates and C₆-C₂₄ alkyl polyglycoside-sulfosuccinates, and salts thereof.

Preferentially, the carboxylate anionic surfactants are chosen, alone or as a mixture, from:
- fatty acids;
- acylglutamates, notably of C₆-C₂₄ or even C₁₂-C₂₀, such as stearoylglutamates, and in particular disodium stearoylglutamate;
- acylsarcosinates, notably of C₆-C₂₄ or even C₁₂-C₂₀, such as palmitoylsarcosinates, and in particular sodium palmitoylsarcosinate;
- acyllactylates, notably of C₁₂-C₂₈ or even C₁₄-C₂₄, such as behenoyllactylates, and in particular sodium behenoyllactylate;
- C₆ -C₂₄ and notably C₁₂ -C₂₀ acylglycinates;
- (C₆-C₂₄)alkyl ether carboxylates, and notably (C₁₂-C₂₀)alkyl ether carboxylates;
- polyoxyalkylenated (C₆-C₂₄)alkyl(amido) ether carboxylic acids, in particular those including from 2 to 50 ethylene oxide groups;
in particular in the form of alkali metal or alkaline-earth metal, ammonium or amino alcohol salts.

Among the above carboxylic surfactants, mention may be made most particularly of surfactants of fatty acid type, notably of C₆-C₃₀. These surfactants are preferably chosen from the compounds of formula (a) below: R-C(O)-OX (a)
with
- X denoting a hydrogen atom, an ammonium ion, an ion derived from an alkali metal or an alkaline-earth metal or an ion derived from an organic amine, preferably a hydrogen atom, and
- R denoting a linear or branched, saturated or unsaturated alkyl group of 7 to 29 carbon atoms.

Preferably, R denotes a linear or branched, saturated or unsaturated alkyl group of 7 to 23 carbon atoms, preferably of 11 to 21 carbon atoms.

Among the fatty acids, mention may be made of lauric acid, palmitic acid, myristic acid, stearic acid, oleic acid and behenic acid.

The fatty acids are advantageously chosen from palmitic acid, myristic acid, stearic acid, and mixtures thereof.

Among the above carboxylic surfactants, mention may be made most particularly of surfactants of sarcosinate type, notably chosen from (C₆-C₃₀)acyl sarcosinates of formula (I) below:

R-C(O)-N(CH₃)-CH₂-C(O)-OX (I)

with
- X denoting a hydrogen atom, an ammonium ion, an ion derived from an alkali metal or an alkaline-earth metal or an ion derived from an organic amine, preferably a hydrogen atom, and
- R denoting a linear or branched alkyl group of 6 to 30 carbon atoms.

Preferably, R denotes a linear or branched alkyl group of 8 to 24 carbon atoms, preferably of 12 to 20 carbon atoms.

Among the (C₆-C₃₀)acyl sarcosinates of formula (I) that may be used in the present composition, mention may be made of palmitoyl sarcosinates, stearoyl sarcosinates, myristoyl sarcosinates, lauroyl sarcosinates and cocoyl sarcosinates, in acid form or in salified form.

The anionic surfactant(s) of sarcosinate type are advantageously chosen from sodium lauroyl sarcosinate, stearoylsarcosine, myristoylsarcosine, and mixtures thereof, preferably from stearoylsarcosine, myristoylsarcosine, and mixtures thereof.

Among the above carboxylic surfactants, mention may also be made of polyoxyalkylenated alkyl(amido) ether carboxylic acids and salts thereof, in particular those including from 2 to 50 alkylene oxide and in particular ethylene oxide groups, such as the compounds sold by the company Kao under the Akypo names.

The polyoxyalkylenated alkyl(amido) ether carboxylic acids that may be used are preferably chosen from those of formula (II):

R₁-(OC₂H₄)ₙ-OCH₂COOA (II)

in which:
- R₁ represents a linear or branched C₆-C₂₄ alkyl or alkenyl radical, a (Cs-C₉)alkylphenyl radical, a radical R₂CONH-CH₂-CH₂- with R₂ denoting a linear or branched C₉-C₂₁ alkyl or alkenyl radical;
   preferably, R₁ is a C₈-C₂₀ and preferably C₈-C₁₈ alkyl radical, and aryl preferably denotes phenyl,
- n is an integer or decimal number (mean value) ranging from 2 to 24 and preferably from 2 to 10,
- A denotes H, ammonium, Na, K, Li, Mg or a monoethanolamine or triethanolamine residue.

Use may also be made of mixtures of compounds of formula (II), in particular mixtures of compounds bearing different groups R₁.

The polyoxyalkylenated alkyl(amido) ether carboxylic acids that are particularly preferred are those of formula (II) in which:
- R₁ denotes a C₁₂-C₁₄ alkyl, cocoyl, oleyl, nonylphenyl or octylphenyl radical,
- A denotes a hydrogen or sodium atom, and
- n ranges from 2 to 20, preferably from 2 to 10.

Even more preferentially, use is made of the compounds of formula (II) in which R₁ denotes a C₁₂ alkyl radical, A denotes a hydrogen or sodium atom and n ranges from 2 to 10.

The sulfonate anionic surfactants that may be used include at least one sulfonate function (-SO₃H or -SO₃⁻).

They may be chosen from the following compounds: alkylsulfonates, alkylamidesulfonates, alkylarylsulfonates, α-olefin sulfonates, paraffin sulfonates, alkyl sulfosuccinates, alkyl ether sulfosuccinates, alkylamidesulfosuccinates, alkylsulfoacetates, N-acyltaurates, acylisethionates; alkylsulfolaurates; and also the salts of these compounds;
the alkyl groups of these compounds including from 6 to 30 carbon atoms, notably from 12 to 28, even better still from 14 to 24 or even from 16 to 22 carbon atoms; the aryl group preferably denoting a phenyl or benzyl group;
these compounds possibly being polyoxyalkylenated, notably polyoxyethylenated, and then preferably including from 1 to 50 ethylene oxide units and better still from 2 to 10 ethylene oxide units.

Preferentially, the sulfonate anionic surfactants are chosen, alone or as a mixture, from:
- C₆-C₂₄ and notably C₁₂-C₂₀ alkyl sulfosuccinates, notably lauryl sulfosuccinates;
- C₆-C₂₄ and notably C₁₂-C₂₀ alkyl ether sulfosuccinates;
- (C₆-C₂₄)acylisethionates, preferably (C₁₂-C₁₈)acylisethionates;
in particular in the form of alkali metal or alkaline-earth metal, ammonium or amino alcohol salts.

The sulfate anionic surfactants that may be used include at least one sulfate function (-OSO₃H or -OSO₃⁻).

They may be chosen from the following compounds: alkyl sulfates, alkyl ether sulfates, alkylamido ether sulfates, alkylaryl polyether sulfates, monoglyceride sulfates; and the salts of these compounds;
the alkyl groups of these compounds including from 6 to 30 carbon atoms, notably from 12 to 28, even better still from 14 to 24 or even from 16 to 22 carbon atoms; the aryl group preferably denoting a phenyl or benzyl group;
these compounds possibly being (poly)oxyalkylenated, notably (poly)oxyethylenated, and then preferably including from 1 to 50 ethylene oxide units and better still from 1 to 10 ethylene oxide units.

Preferentially, the sulfate anionic surfactants are chosen, alone or as a mixture, from:
- alkyl sulfates, notably C₆-C₂₄ or even C₁₂-C₂₀ alkyl sulfates;
- alkyl ether sulfates, notably C₆-C₂₄ or even C₁₂-C₂₀ alkyl ether sulfates, preferably comprising from 1 to 20 ethylene oxide units;
in particular in the form of alkali metal or alkaline-earth metal, ammonium or amino alcohol salts.

When the anionic surfactant is in salt form, said salt may be chosen from alkali metal salts, such as the sodium or potassium salt, ammonium salts, amine salts and in particular amino alcohol salts, and alkaline-earth metal salts, such as the magnesium salt.

Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

Preferentially, the anionic surfactant(s) are chosen from:
- C₆-C₃₀, notably C₈-C₂₄ fatty acids;
- C₆-C₂₄ and notably C₁₂-C₂₀ alkyl sulfates;
- C₆-C₂₄ and notably C₁₂-C₂₀ alkyl ether sulfates; preferably comprising from 1 to 20 ethylene oxide units;
- C₆-C₂₄ alkyl sulfosuccinates and notably C₁₂-C₂₀ alkyl sulfosuccinates; notably lauryl sulfosuccinates;
- C₆-C₂₄ and notably C₁₂-C₂₀ alkyl ether sulfosuccinates;
- (C₆-C₂₄)acylisethionates, preferably (C₁₂-C₁₈)acylisethionates;
- C₆-C₂₄ and notably C₁₂-C₂₀ acylsarcosinates; notably palmitoylsarcosinates, stearoylsarcosinates, myristoylsarcosinates;
- (C₆-C₂₄)alkyl ether carboxylates, preferably (C₁₂-C₂₀)alkyl ether carboxylates;
- polyoxyalkylenated (C₆-C₂₄)alkyl(amido) ether carboxylic acids and salts thereof, in particular those including from 2 to 50 alkylene oxide groups and in particular ethylene oxide groups;
- C₆-C₂₄ and notably C₁₂-C₂₀ acylglutamates;
- C₆ -C₂₄ and notably C₁₂ -C₂₀ acylglycinates;
- and also salts thereof, in particular the alkali metal or alkaline-earth metal or zinc, ammonium or amino alcohol salts thereof;
- and mixtures thereof.

Advantageously, the anionic surfactant(s) are chosen from carboxylate anionic surfactants, and mixtures thereof.

The anionic surfactant(s) are preferably chosen from C₆-C₃₀ fatty acids, acyl(C₆-C₃₀)glycinates, acyl(C₆-C₃₀)lactylates, acyl(C₆-C₃₀)sarcosinates, acyl(C₆-C₃₀)glutamates; alkyl-D-galactosiduronic acids, alkyl ether carboxylic acids, alkyl(C₆-C₃₀ aryl) ether carboxylic acids, alkylamido ether carboxylic acids; monoesters of C₆-C₂₄ alkyl and of polyglycoside-polycarboxylic acids; and also the salts of these compounds; and mixtures thereof.

The anionic surfactant(s) are advantageously chosen from (C₆-C₂₄)acyl sarcosinates, C₆-C₂₄ fatty acids and mixtures thereof, preferably from stearoylsarcosine, myristoylsarcosine, myristic acid, stearic acid, palmitic acid, salts thereof and mixtures thereof.

Advantageously, the total content of the anionic surfactant(s) is greater than or equal to 0.05% by weight, preferentially greater than or equal to 0.1% by weight, relative to the total weight of the composition.

Preferably, the total content of the anionic surfactant(s) ranges from 0.1% to 5% by weight, more preferentially from 0.2% to 1% by weight, relative to the total weight of the composition.

Preferably, the total content of the carboxylate anionic surfactant(s) ranges from 0.1% to 5% by weight, more preferentially from 0.2% to 1% by weight, relative to the total weight of the composition.

### Fatty alcohols

The composition contained in the aerosol device according to the invention also comprises one or more fatty alcohols.

The term "fatty alcohol" means a long-chain aliphatic alcohol comprising from 6 to 40 carbon atoms, preferably from 8 to 40 carbon atoms, and comprising at least one hydroxyl group OH.

The fatty alcohols according to the invention are preferably non-oxyalkylenated and non-glycerolated.

The fatty alcohols according to the invention may be saturated or unsaturated, and linear or branched, and preferably include from 8 to 40 carbon atoms.

More preferentially, the fatty alcohols according to the invention are chosen from compounds having the structure R-OH with R denoting a linear or branched, saturated or unsaturated alkyl group optionally substituted with one or more hydroxyl groups, comprising from 8 to 40, better still from 10 to 30, or even from 12 to 24 and even better still from 14 to 22 carbon atoms.

The fatty alcohols may be chosen from solid fatty alcohols and liquid fatty alcohols, and mixtures thereof.

For the purposes of the present invention, the term "solid fatty alcohol" means a fatty alcohol with a melting point of greater than 25°C, preferably greater than or equal to 28°C, more preferentially greater than or equal to 30°C at atmospheric pressure (1.013×10⁵ Pa).

The solid fatty alcohols may be chosen from saturated or unsaturated, linear or branched solid fatty alcohols, including from 8 to 40 carbon atoms.

The solid fatty alcohols that may be used according to the invention are preferably chosen from compounds having the structure R-OH with R denoting a saturated linear alkyl group optionally substituted with one or more hydroxyl groups, comprising from 8 to 40, better still from 10 to 30, or even from 12 to 24 and even better still from 14 to 22 carbon atoms.

The solid fatty alcohols that may be used may be chosen, alone or as a mixture, from:
- lauryl alcohol (or 1-dodecanol);
- myristyl alcohol (or 1-tetradecanol);
- cetyl alcohol (or 1-hexadecanol);
- stearyl alcohol (or 1-octadecanol);
- arachidyl alcohol (or 1-eicosanol);
- behenyl alcohol (or 1-docosanol);
- lignoceryl alcohol (or 1-tetracosanol);
- ceryl alcohol (or 1-hexacosanol);
- montanyl alcohol (or 1-octacosanol);
- myricyl alcohol (or 1-triacontanol).

Preferentially, the fatty alcohol(s) are chosen from cetyl alcohol, stearyl alcohol, behenyl alcohol, myristyl alcohol, and mixtures thereof, such as cetylstearyl alcohol or cetearyl alcohol.

For the purposes of the present invention, the term "liquid fatty alcohol" means a fatty alcohol with a melting point of less than or equal to 25°C, preferably less than or equal to 20°C at atmospheric pressure (1.013×10⁵ Pa).

The liquid fatty alcohols that may be used according to the invention are preferably chosen from compounds having the structure R-OH with R denoting a saturated or unsaturated, linear or branched, preferably unsaturated and/or branched, alkyl group optionally substituted with one or more hydroxyl groups, comprising from 8 to 40, better still from 10 to 30, or even from 12 to 24 and even better still from 14 to 22 carbon atoms.

The liquid fatty alcohols that may be used may be chosen, alone or as a mixture, from oleyl alcohol, linoleyl alcohol, linolenyl alcohol, isocetyl alcohol, isostearyl alcohol, 2-octyl-1-dodecanol, 2-butyloctanol, 2-hexyl-1-decanol, 2-decyl-1-tetradecanol and 2-tetradecyl-1-cetanol, and mixtures thereof.

The fatty alcohol(s) according to the invention are advantageously chosen from solid fatty alcohols, and mixtures thereof, preferably from saturated linear solid fatty alcohols including from 8 to 40 carbon atoms, and mixtures thereof.

Preferably, the fatty alcohol(s) are chosen from cetyl alcohol, stearyl alcohol, behenyl alcohol and myristyl alcohol, and mixtures thereof.

Advantageously, the total content of the fatty alcohol(s) ranges from 0.05% to 10% by weight, preferably from 0.1% to 8% by weight, more preferentially from 1% to 5% by weight, relative to the total weight of the composition.

The weight ratio R between the total content of the fatty alcohol(s) and the total content of the anionic surfactant(s) ranges from 1.5 to 10. This weight ratio R preferably ranges from 2 to 8, more preferentially from 3 to 7, even more preferentially from 4 to 6.

In one particular variant of the invention, the weight ratio between the total content of the solid fatty alcohol(s) and the total content of the carboxylate anionic surfactant(s) ranges from 1.5 to 10, preferably from 2 to 8, more preferentially from 3 to 7 and even more preferentially from 4 to 6.

### Propellants

The composition contained in the aerosol device according to the invention also comprises one or more propellants chosen from compressed gases.

For the purposes of the present invention, the term "compressed gases" means gases that are not liquified when compressed in the aerosol device of the invention, usually between 6 and 10 bars, at room temperature (25°C).

The propellants that may be used in the aerosol device of the present invention are advantageously chosen from air, nitrogen, carbon dioxide, and mixtures thereof, preferably carbon dioxide.

The composition may optionally comprise one or more propellants other than compressed gases, notably chosen from liquefied gases and mixtures thereof, more preferentially dimethyl ether, chlorinated and/or fluorinated hydrocarbons, such as trichlorofluoromethane, dichlorodifluoromethane, chlorodifluoromethane, 1,1,1,2-tetrafluoroethane, chloropentafluoroethane, 1-chloro-1,1-difluoroethane, 1,1-difluoroethane, 1,3,3,3-tetrafluoroprop-1-ene; volatile hydrocarbons, which are optionally halogenated, notably such as C₃ to C₅ alkanes, for instance propane, isopropane, n-butane, isobutane, pentane, and mixtures thereof, more preferentially from C₃ to C₅ alkanes, and mixtures thereof, and better still from propane, isopropane, n-butane, isobutane, and mixtures thereof.

Advantageously, the total content of the propellant(s) ranges from 0.5% to 10% by weight, preferably from 1% to 5% by weight, more preferentially from 1.5% to 4% by weight, relative to the total weight of the composition.

Advantageously, the total content of the compressed gas(es) ranges from 0.5% to 10% by weight, preferably from 1% to 5% by weight, more preferentially from 1.5% to 4% by weight, relative to the total weight of the composition.

In a preferred variant of the invention, the content of carbon dioxide ranges from 0.5% to 10% by weight, preferably from 1% to 5% by weight, more preferentially from 1.5% to 4% by weight, relative to the total weight of the composition.

### Fatty substances other than fatty alcohols

The cosmetic composition contained in the aerosol device according to the present invention may optionally also comprise one or more fatty substances, preferably one or more liquid fatty substances, other than the fatty alcohols of the invention.

The liquid fatty substances are generally soluble in organic solvents under the same temperature and pressure conditions, for instance chloroform, ethanol, benzene, liquid petroleum jelly or decamethylcyclopentasiloxane.

For the purposes of the present invention, the term "liquid fatty substance" means a fatty substance with a melting point of less than or equal to 25°C, preferably less than or equal to 20°C at atmospheric pressure (1.013×10⁵ Pa).

They preferably have a viscosity of less than or equal to 2 Pa.s, better still less than or equal to 1 Pa.s, and even better still less than or equal to 0.1 Pa.s at a temperature of 25°C and at a shear rate of 1 s⁻¹.

The liquid fatty substances that may be used in the composition according to the invention are generally not oxyalkylenated and preferably do not contain any carboxylic acid COOH functions.

Advantageously, the liquid fatty substances according to the invention may be chosen from hydrocarbons, fatty esters preferably comprising from 8 to 40 carbon atoms, fatty ethers preferably comprising from 8 to 40 carbon atoms, silicones and mixtures thereof.

The term "liquid hydrocarbon" means a hydrocarbon composed solely of carbon and hydrogen atoms, which is liquid at a temperature of 25°C and at atmospheric pressure (1.013 × 10⁵ Pa), of mineral, plant or synthetic origin.

More particularly, the liquid hydrocarbons are chosen from:
- linear or branched, optionally cyclic, C₆-C₁₆ alkanes. Examples that may be mentioned include hexane, undecane, dodecane, tridecane, and isoparaffins, for instance isohexadecane, isododecane and isodecane, and mixtures thereof.
- linear or branched hydrocarbons of mineral, animal or synthetic origin, of more than 16 carbon atoms, such as liquid paraffin or liquid petroleum jelly, polydecenes, hydrogenated polyisobutene such as the product sold under the brand name Parleam^{®} by the company NOF Corporation, and squalane.

In one preferred variant, the liquid hydrocarbon(s) are chosen from linear or branched, optionally cyclic, C₆-C₁₆ alkanes.

The term "liquid fatty ester" means an ester derived from a fatty acid and/or from a fatty alcohol, which is liquid at 25°C and at atmospheric pressure (1.013×10⁵ Pa) and which is different from the monoester(s) of a fatty acid and of (poly)glyceryl.

The liquid fatty esters are preferably liquid esters of saturated or unsaturated linear or branched C₁-C₂₆ aliphatic mono- or polyacids and of saturated or unsaturated linear or branched C₁-C₂₆ aliphatic mono- or polyalcohols, the total number of carbon atoms in the the liquid esters being greater than or equal to 10.

Preferably, for the fatty esters of monoalcohols, at least one from among the alcohol or the acid from which the esters of the invention are derived is branched.

Among the monoesters of monoacids and of monoalcohols, mention may be made of ethyl palmitate, isopropyl palmitate, alkyl myristates such as isopropyl myristate or ethyl myristate, isocetyl stearate, ethyl laurate, 2-ethylhexyl isononanoate, isononyl isononanoate, ethyl octanoate, ethyl caprate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy non-sugar alcohols may also be used.

Mention may be made notably of diethyl sebacate, diisopropyl sebacate, bis(2-ethylhexyl) sebacate, diisopropyl adipate, di-n-propyl adipate, dioctyl adipate, bis(2-ethylhexyl) adipate, diisostearyl adipate, bis(2-ethylhexyl) maleate, triisopropyl citrate, triisocetyl citrate, triisostearyl citrate, glyceryl trilactate, glyceryl trioctanoate, trioctyldodecyl citrate, trioleyl citrate, neopentyl glycol diheptanoate, and diethylene glycol diisononanoate.

The composition may also comprise, as liquid fatty ester, sugar esters and diesters of C₆-C₃₀ and preferably C₁₂-C₂₂ fatty acids. It is recalled that the term "sugar" refers to oxygen-bearing hydrocarbon-based compounds containing several alcohol functions, with or without aldehyde or ketone functions, and which include at least 4 carbon atoms. These sugars may be monosaccharides, oligosaccharides or polysaccharides.

Examples of suitable sugars that may be mentioned include saccharose, glucose, galactose, ribose, fucose, maltose, fructose, mannose, arabinose, xylose and lactose, and derivatives thereof, notably alkyl derivatives, such as methyl derivatives, for instance methylglucose.

The sugar esters of fatty acids may be notably chosen from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

These esters may be, for example, oleates, laurates, palmitates, myristates, behenates, cocoates, stearates, linoleates, linolenates, caprates and arachidonates, and mixtures thereof, notably such as oleopalmitate, oleostearate or palmitostearate mixed esters.

More particularly, use is made of monoesters and diesters and notably of saccharose, glucose or methylglucose mono- or dioleates, stearates, behenates, oleopalmitates, linoleates, linolenates or oleostearates.

An example that may be mentioned is the product sold under the name Glucate^{®} DO by the company Amerchol, which is a methylglucose dioleate.

Finally, use may also be made of natural or synthetic esters of di- or triacids with glycerol.

Among these, mention may be made of plant oils or oils of plant origin.

As oils of plant origin or synthetic triglycerides that can be used in the composition of the invention as liquid fatty esters, examples that may be mentioned include triglyceride oils of plant or synthetic origin, such as liquid fatty acid triglycerides including from 6 to 30 carbon atoms, for instance heptanoic or octanoic acid triglycerides, or alternatively, for example, sunflower oil, corn oil, soya bean oil, marrow oil, grapeseed oil, sesame oil, hazelnut oil, apricot oil, macadamia oil, arara oil, castor oil, avocado oil, olive oil, rapeseed oil, coconut kernel oil, wheatgerm oil, sweet almond oil, apricot oil, safflower oil, candlenut oil, camelina oil, tamanu oil, babassu oil and pracaxi oil, caprylic/capric acid triglycerides, for instance those sold by the company Stéarinerie Dubois or those sold under the names Miglyol^{®} 810, 812 and 818 by the company Dynamit Nobel, jojoba oil and shea butter oil.

Preferably, use will be made, as esters according to the invention, of liquid fatty esters derived from monoalcohols, in particular isopropyl myristate or palmitate, or natural or synthetic esters of di- or triacids with glycerol, in particular plant oils.

The liquid fatty ethers may be chosen from liquid dialkyl ethers such as dicaprylyl ether.

The oil(s) that can be used in the composition according to the invention may also be chosen from silicones.

Preferably, the liquid silicone(s) are chosen from polydialkylsiloxanes, notably polydimethylsiloxanes (PDMS), and organomodified polysiloxanes including at least one functional group chosen from amino groups, aryl groups and alkoxy groups.

Organopolysiloxanes are defined in greater detail in Walter Noll's *Chemistry and Technology of Silicones* (1968), Academic Press. They may be volatile or non-volatile.

The liquid non-volatile silicones that may be used in the composition according to the invention may preferably be liquid non-volatile polydialkylsiloxanes, polyorganosiloxanes modified with organofunctional groups chosen from amine groups, aryl groups and alkoxy groups, and also mixtures thereof.

These silicones are more particularly chosen from polydialkylsiloxanes, among which mention may be made mainly of polydimethylsiloxanes bearing trimethylsilyl end groups. The viscosity of the silicones is measured at 25°C according to ASTM standard 445 Appendix C.

Among these polydialkylsiloxanes, mention may be made, in a non-limiting manner, of the following commercial products:
- the Silbione^{®} oils of the 47 and 70 047 series or the Mirasil^{®} oils sold by Rhodia;
- the oils of the Mirasil^{®} series sold by the company Rhodia;
- the oils of the 200 series from Dow Corning;
- the Viscasil^{®} oils from General Electric and certain oils of the SF series (SF 96, SF 18) from General Electric.

Mention may also be made of polydimethylsiloxanes bearing dimethylsilanol end groups, known under the name dimethiconol (CTFA), such as the oils of the 48 series from the company Rhodia.

The organomodified silicones that may be used in accordance with the invention are silicones as defined above and including in their structure one or more organofunctional groups attached via a hydrocarbon-based group.

The organomodified silicones may be polydiarylsiloxanes, notably polydiphenylsiloxanes, and polyalkylarylsiloxanes functionalized with the organofunctional groups mentioned previously.

The polyalkylarylsiloxanes are particularly chosen from linear and/or branched polydimethyl/methylphenylsiloxanes and polydimethyl/diphenylsiloxanes with a viscosity at 25°C and atmospheric pressure (1.013 × 10⁵ Pa) ranging from 1×10⁻⁵ to 5×10⁻² m²/s at 25°C.

Among these polyalkylarylsiloxanes, examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Among the organomodified silicones, mention may also be made of polyorganosiloxanes including:
- substituted or unsubstituted amine groups, for instance the products sold under the names GP 4 Silicone Fluid and GP 7100 by Genesee. The substituted amino groups are, in particular, C₁-C₄ aminoalkyl groups;
- alkoxylated groups, such as the product sold under the name Silicone Copolymer F-755 by SWS Silicones and Abil Wax^{®} 2428, 2434 and 2440 by the company Goldschmidt.

The volatile silicones are more particularly chosen from those with a boiling point of between 60°C and 260°C, and even more particularly from:
- cyclic silicones including from 3 to 7 and preferably 4 to 6 silicon atoms.

These are, for example, octamethylcyclotetrasiloxane sold notably under the name Volatile Silicone 7207 by the company Union Carbide or Silbione 70045 V 2 by the company Rhodia, decamethylcyclopentasiloxane sold under the name Volatile Silicone 7158 by the company Union Carbide, and Silbione 70045 V 5 by the company Rhodia, and mixtures thereof.

Mention may also be made of cyclocopolymers of the dimethylsiloxane/methylalkylsiloxane type, such as Volatile Silicone FZ 3109 sold by the company Union Carbide, of chemical structure:

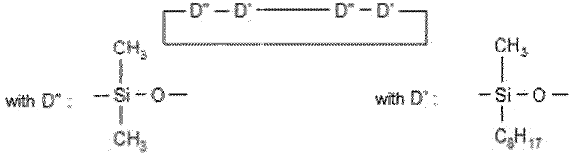

Mention may also be made of mixtures of cyclic silicones with organosilicon compounds, such as the mixture of octamethylcyclotetrasiloxane and tetrakis(trimethylsilyl)pentaerythritol (50/50) and the mixture of octamethylcyclotetrasiloxane and oxy-1,1'-bis(2,2,2',2',3,3'-hexatrimethylsilyloxy)neopentane;
- linear volatile silicones containing 2 to 9 silicon atoms and having a viscosity of less than or equal to 5×10⁻⁶ m²/s at 25°C. Examples include hexamethyldisiloxane, octamethyltrisiloxane and decamethyltetrasiloxane sold notably under the name SH 200 by the company Toray Silicone. Silicones belonging to this category are also described in the article published in Cosmetics and Toiletries, Vol. 91, Jan. 76, pages 27-32 - Todd & Byers Volatile Silicone Fluids for Cosmetics*.* Preferably, the linear volatile silicones contain from 2 to 7 silicon atoms and better still from 3 to 6 silicon atoms.

Preferably, the volatile silicones are chosen from cyclic silicones including from 4 to 6 silicon atoms and linear silicones containing 4 to 6 silicon atoms.

The silicones may also be chosen from amino silicones.

The term "amino silicone" denotes any silicone including at least one primary, secondary or tertiary amine or a quaternary ammonium group.

The weight-average molecular masses of these amino silicones may be measured by gel permeation chromatography (GPC) at room temperature (25°C), as polystyrene equivalent. The columns used are µ styragel columns. The eluent is THF and the flow rate is 1 ml/min. 200 µl of a 0.5% by weight solution of silicone in THF are injected. Detection is performed by refractometry and UV-metry.

Preferably, the amino silicone(s) that may be used in the context of the invention are chosen from:
a) the polysiloxanes corresponding to formula (A): 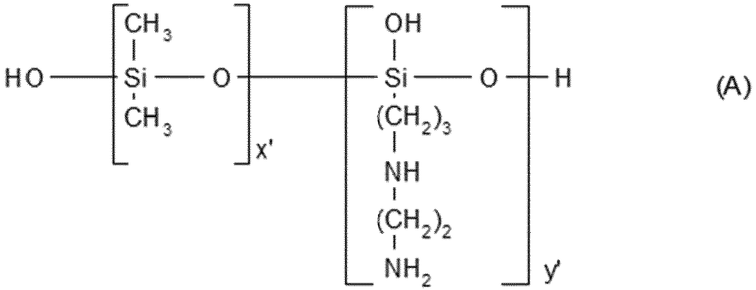 in which x' and y' are integers such that the weight-average molecular weight (Mw) is between 5000 and 500 000 approximately;
b) the amino silicones corresponding to formula (B):

   R'ₐG₃₋ₐ-Si(OSiG₂)ₙ-(OSiG_{b}R'_{2-b})ₘ-O-SiG₃₋ₐ-R'ₐ (B)

   in which:
   - G, which may be identical or different, denotes a hydrogen atom or a phenyl, OH, C₁-C₈ alkyl, for example methyl, or C₁-C₈ alkoxy, for example methoxy, group,
   - a, which may be identical or different, denotes 0 or an integer from 1 to 3, in particular 0,
   - b denotes 0 or 1, in particular 1,
   - m and n are numbers such that the sum (n + m) ranges from 1 to 2000 and in particular from 50 to 150, n possibly denoting a number from 0 to 1999 and notably from 49 to 149, and m possibly denoting a number from 1 to 2000 and notably from 1 to 10;
   - R', which may be identical or different, denotes a monovalent radical of formula -CqH2qL in which q is a number ranging from 2 to 8 and L is an optionally quaternized amine group chosen from the following groups:
      - N(R")₂; -N⁺(R")₃ A-; -NR"-Q-N(R")₂ and -NR"-Q-N⁺(R")₃ A-,
   in which R", which may be identical or different, denotes hydrogen, phenyl, benzyl, or a saturated monovalent hydrocarbon-based radical, for example a C1-C20 alkyl radical; Q denotes a linear or branched group of formula CᵣH₂ᵣ, r being an integer ranging from 2 to 6, preferably from 2 to 4; and A- represents a cosmetically acceptable anion, notably a halide such as fluoride, chloride, bromide or iodide.

Preferably, the amino silicones are chosen from the amino silicones of formula (B). Preferably, the amino silicones of formula (B) are chosen from the amino silicones corresponding to formulae (C), (D), (E), (F) and/or (G) below.

According to a first embodiment, the amino silicones corresponding to formula (B) are chosen from the silicones known as "trimethylsilyl amodimethicone" corresponding to formula (C): 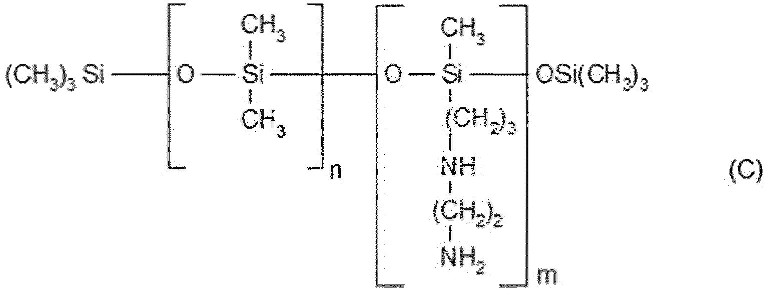 in which m and n are numbers such that the sum (n + m) ranges from 1 to 2000 and in particular from 50 to 150, it being possible for n to denote a number from 0 to 1999 and notably from 49 to 149, and for m to denote a number from 1 to 2000 and notably from 1 to 10.

According to a second embodiment, the amino silicones corresponding to formula (B) are chosen from the silicones of formula (D) below: 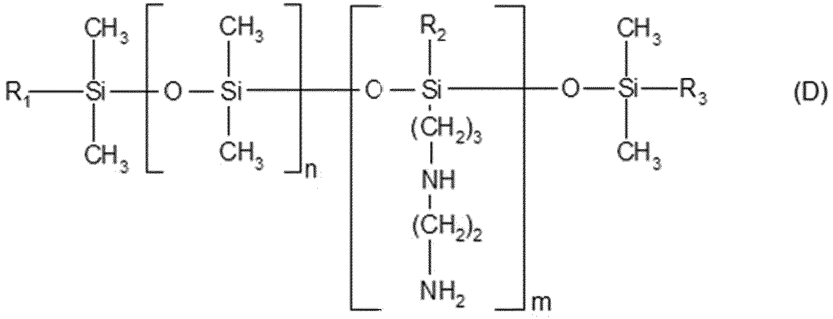 in which:
- m and n are numbers such that the sum (n + m) ranges from 1 to 1000 and in particular from 50 to 250 and more particularly from 100 to 200; it being possible for n to denote a number from 0 to 999 and notably from 49 to 249 and more particularly from 125 to 175, and for m to denote a number from 1 to 1000 and notably from 1 to 10, and more particularly from 1 to 5;
- R₁, R₂ and R₃, which may be identical or different, represent a hydroxyl or C₁-C₄ alkoxy radical, at least one of the radicals R₁ to R₃ denoting an alkoxy radical.

Preferably, the alkoxy radical is a methoxy radical.

The hydroxy/alkoxy mole ratio preferably ranges from 0.2:1 to 0.4:1 and preferably from 0.25:1 to 0.35:1 and more particularly equals 0.3:1.

The weight-average molecular mass (Mw) of these silicones preferably ranges from 2000 to 1 000 000 and more particularly from 3500 to 200 000.

According to a third embodiment, the amino silicones corresponding to formula (B) are chosen from the silicones of formula (E) below: 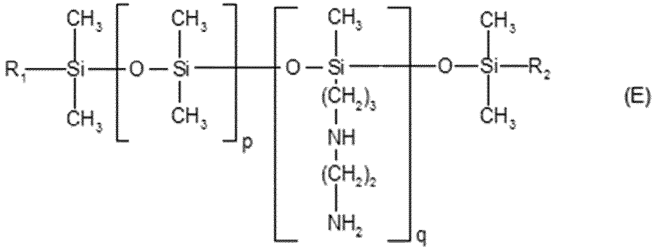 in which:
- p and q are numbers such that the sum (p + q) ranges from 1 to 1000, in particular from 50 to 350 and more particularly from 150 to 250; it being possible for p to denote a number from 0 to 999 and notably from 49 to 349 and more particularly from 159 to 239, and for q to denote a number from 1 to 1000, notably from 1 to 10 and more particularly from 1 to 5;
- R₁ and R₂, which may be different, represent a hydroxyl or C₁-C₄ alkoxy radical, at least one of the radicals R₁ or R₂ denoting an alkoxy radical.

Preferably, the alkoxy radical is a methoxy radical.

The hydroxy/alkoxy mole ratio generally ranges from 1:0.8 to 1:1.1 and preferably from 1:0.9 to 1:1 and more particularly equals 1:0.95.

The weight-average molecular mass (Mw) of the silicone preferably ranges from 2000 to 200 000, even more particularly from 5000 to 100 000 and more particularly from 10 000 to 50 000.

The commercial products comprising silicones of structure (D) or (E) may include in their composition one or more other amino silicones the structure of which is different from formula (D) or (E).

A product containing amino silicones of structure (D) is sold by the company Wacker under the name Belsil^{®} ADM 652.

A product containing amino silicones of structure (E) is sold by Wacker under the name Fluid WR 1300^{®}.

When these amino silicones are used, one particularly advantageous embodiment consists in using them in the form of an oil-in-water emulsion. The oil-in-water emulsion may comprise one or more surfactants. The surfactants may be of any nature but are preferably cationic and/or nonionic. The number-average size of the silicone particles in the emulsion generally ranges from 3 nm to 500 nanometres. Preferably, notably as amino silicones of formula (E), use is made of microemulsions with a mean particle size ranging from 5 nm to 60 nanometres (limits included) and more particularly from 10 nm to 50 nanometres (limits included). Thus, use may be made according to the invention of the amino silicone microemulsions of formula (E) sold under the names Finish CT 96 E^{®} or SLM 28020^{®} by the company Wacker.

According to a fourth embodiment, the amino silicones corresponding to formula (B) are chosen from the silicones of formula (F) below: 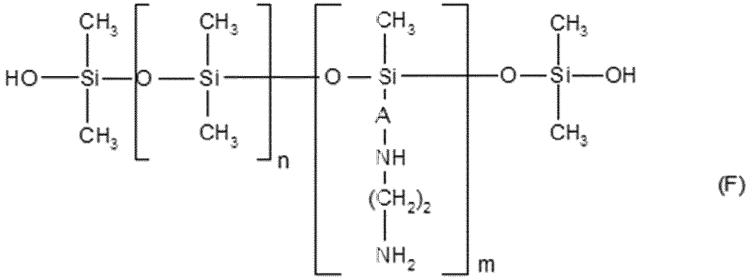 in which:
- m and n are numbers such that the sum (n + m) ranges from 1 to 2000 and in particular from 50 to 150, it being possible for n to denote a number from 0 to 1999 and notably from 49 to 149, and for m to denote a number from 1 to 2000 and notably from 1 to 10;
- A denotes a linear or branched alkylene radical containing from 4 to 8 carbon atoms and preferably 4 carbon atoms. This radical is preferably linear.

The weight-average molecular mass (Mw) of these amino silicones preferably ranges from 2000 to 1 000 000 and even more particularly from 3500 to 200 000.

A silicone corresponding to this formula is, for example, the Xiameter MEM 8299 Emulsion from Dow Corning.

According to a fifth embodiment, the amino silicones corresponding to formula (B) are chosen from the silicones of formula (G) below: 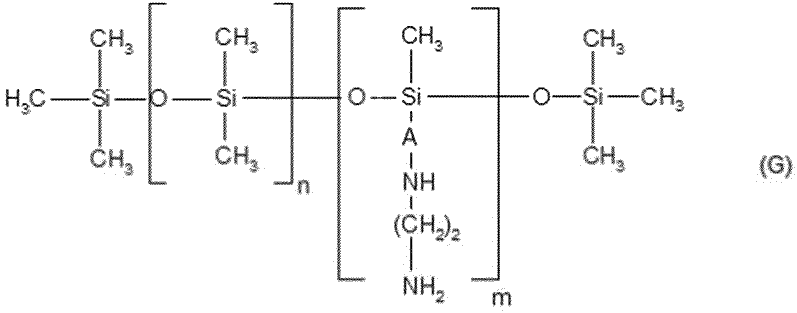 in which:
- m and n are numbers such that the sum (n + m) ranges from 1 to 2000 and in particular from 50 to 150, it being possible for n to denote a number from 0 to 1999 and notably from 49 to 149, and for m to denote a number from 1 to 2000 and notably from 1 to 10;
- A denotes a linear or branched alkylene radical containing from 4 to 8 carbon atoms and preferably 4 carbon atoms. This radical is preferably branched.

The weight-average molecular mass (Mw) of these amino silicones preferably ranges from 500 to 1 000 000 and even more particularly from 1000 to 200 000.

A silicone corresponding to this formula is, for example, DC2-8566 Amino Fluid from Dow Corning;
c) the amino silicones corresponding to formula (H): 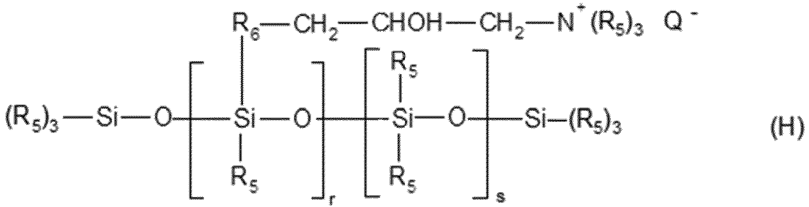 in which:
- R₅ represents a monovalent hydrocarbon-based radical containing from 1 to 18 carbon atoms, and in particular a C₁-C₁₈ alkyl or C₂-C₁₈ alkenyl, for example methyl, radical;
- R₆ represents a divalent hydrocarbon-based radical, notably a C₁-C₁₈ alkylene radical or a divalent C₁-C₁₈, for example C₁-C₈, alkyleneoxy radical linked to the Si via an SiC bond;
- Q- is an anion such as a halide, notably chloride, ion or an organic acid salt, notably acetate;
- r represents a mean statistical value ranging from 2 to 20 and in particular from 2 to 8;
- s represents a mean statistical value ranging from 20 to 200 and in particular from 20 to 50.

Such amino silicones are notably described in patent US 4 185 087;
d) the silicones comprising a quaternary ammonium having the formula below: 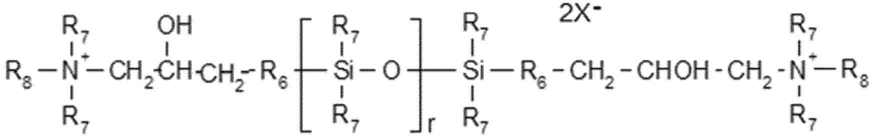 in which:
   - R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring comprising 5 or 6 carbon atoms, for example methyl;
   - R₆ represents a divalent hydrocarbon-based radical, notably a C₁-C₁₈ alkylene radical or a divalent C₁-C₁₈, for example C₁-C₈, alkyleneoxy radical linked to the Si via an SiC bond;
   - R₈, which may be identical or different, represent a hydrogen atom, a monovalent hydrocarbon-based radical containing from 1 to 18 carbon atoms, and in particular a C₁-C₁₈ alkyl radical, a C₂-C₁₈ alkenyl radical or a radical -R₆-NHCOR₇;
   - X- is an anion such as a halide, notably chloride, ion or an organic acid salt, notably acetate;
   - r represents a mean statistical value ranging from 2 to 200 and in particular from 5 to 100.
   These silicones are described, for example, in patent application EP-A 0 530 974;
e) the amino silicones of formula (J): 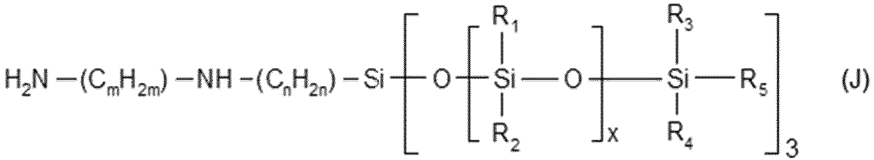 in which:
   - R₁, R₂, R₃ and R₄, which may be identical or different, denote a C₁-C₄ alkyl radical or a phenyl group,
   - R₅ denotes a C₁-C₄ alkyl radical or a hydroxyl group,
   - n is an integer ranging from 1 to 5,
   - m is an integer ranging from 1 to 5, and
   - x is chosen such that the amine number ranges from 0.01 to 1 meq/g;
f) the multiblock polyoxyalkylenated amino silicones, of the type (AB)n, A being a polysiloxane block and B being a polyoxyalkylenated block including at least one amine group.

Said silicones are preferably constituted of repeating units of the following general formulae:

[-(SiMe₂O)ₓSiMe₂ - R -N(R")- R'-O(C₂H₄O)ₐ(C₃H₆O)_{b} -R'-N(H)-R-]

or alternatively

[-(SiMe₂O)ₓSiMe₂ - R -N(R")- R' - O(C₂H₄O)ₐ(C₃H₆O)_{b} -]

in which:
- a is an integer greater than or equal to 1, preferably ranging from 5 to 200 and more particularly ranging from 10 to 100;
- b is an integer between 0 and 200, preferably ranging from 4 to 100 and more particularly between 5 and 30;
- x is an integer ranging from 1 to 10 000 and more particularly from 10 to 5000;
- R" is a hydrogen atom or a methyl;
- R, which may be identical or different, represent a linear or branched divalent C₂-C₁₂ hydrocarbon-based radical, optionally including one or more heteroatoms such as oxygen; preferably, R denotes an ethylene radical, a linear or branched propylene radical, a linear or branched butylene radical or a radical - CH₂CH₂CH₂OCH₂CH(OH)CH₂-; preferentially, R denotes a radical - CH₂CH₂CH₂OCH₂CH(OH)CH₂-;
- R', which may be identical or different, represent a linear or branched divalent C₂-C₁₂ hydrocarbon-based radical, optionally including one or more heteroatoms such as oxygen; preferably, R' denotes an ethylene radical, a linear or branched propylene radical, a linear or branched butylene radical or a radical - CH₂CH₂CH₂OCH₂CH(OH)CH₂-; preferentially, R' denotes -CH(CH₃)-CH₂-.

The siloxane blocks preferably represent between 50 mol% and 95 mol% of the total weight of the silicone, more particularly from 70 mol% to 85 mol%.

The amine content is preferably between 0.02 and 0.5 meq/g of copolymer in a 30% solution in dipropylene glycol, more particularly between 0.05 and 0.2.

The weight-average molecular mass (Mw) of the silicone is preferably between 5000 and 1 000 000 and more particularly between 10 000 and 200 000.

Mention may be made notably of the silicones sold under the names Silsoft A-843 or Silsoft A+ by Momentive;
g) and mixtures thereof.

When the composition according to the invention comprises one or more fatty substances other than fatty alcohols, their total content preferably ranges from 0.05% to 10% by weight and preferentially from 0.1% to 5% by weight, relative to the total weight of the composition.

### Fixing polymers

The cosmetic composition contained in the aerosol device according to the present invention may optionally also comprise one or more fixing polymers.

For the purposes of the invention, the term "fixing polymer" means any polymer that is capable, by application to the hair, of giving a shape to the head of hair or of holding an already acquired shape.

All the anionic, cationic, amphoteric and nonionic fixing polymers and mixtures thereof used in the art may be used in the compositions according to the present application.

Preferably, the fixing polymers are chosen from nonionic fixing polymers.

The anionic fixing polymers generally used are polymers including groups derived from carboxylic, sulfonic or phosphoric acid, and have a number-average molecular mass of between about 500 and 5 000 000.

The carboxylic groups are provided by unsaturated mono- or dicarboxylic acid monomers, such as those corresponding to formula (III): 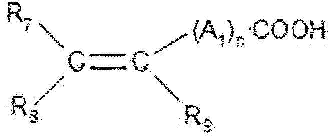 in which n is an integer from 0 to 10, A1 denotes a methylene group, optionally connected to the carbon atom of the unsaturated group or to the neighbouring methylene group when n is greater than 1 via a heteroatom such as oxygen or sulfur, R₇ denotes a hydrogen atom or a phenyl or benzyl group, R₈ denotes a hydrogen atom or a lower alkyl or carboxyl group, and R₉ denotes a hydrogen atom, a lower alkyl group or a -CH₂-COOH, phenyl or benzyl group.

In the abovementioned formula, a lower alkyl group preferably denotes a group containing 1 to 4 carbon atoms and in particular methyl and ethyl groups.

The anionic fixing polymers containing carboxylic groups that are preferred according to the invention are:
A) copolymers of acrylic or methacrylic acid or salts thereof.
   Among these polymers, mention may be made of copolymers of acrylic or methacrylic acid with a monoethylenic monomer, such as ethylene, styrene, vinyl esters or acrylic or methacrylic acid esters, optionally grafted to a polyalkylene glycol, such as polyethylene glycol, and optionally crosslinked. Such polymers are described in particular in French patent No.1 222 944 and German patent application No. 2 330 956, the copolymers of this type including an optionally N-alkylated and/or hydroxyalkylated acrylamide unit in their chain as described notably in Luxembourg patent application Nos. 75370 and 75371. Mention may also be made of copolymers of acrylic acid and of C₁-C₄ alkyl methacrylate and terpolymers of vinylpyrrolidone, of acrylic acid and of C₁-C₂₀ alkyl methacrylate, for example lauryl methacrylate, such as that sold by the company ISP under the name Acrylidone^{®} LM (INCI name: VP/acrylates/lauryl methacrylate copolymer), acrylic acid/ethyl acrylate/N-(t-butyl)acrylamide terpolymers, such as the products Ultrahold^{®} Strong and Ultrahold^{®} 8 sold by the company BASF (INCI name: Acrylates/t-butylacrylamide copolymer), methacrylic acid/ethyl acrylate/tert-butyl acrylate terpolymers, such as the products sold under the names Luvimer^{®} 100 P or Luvimer^{®} PRO 55 by the company BASF (INCI name: Acrylates copolymer), copolymers of methacrylic acid and of ethyl acrylate, such as the products sold under the names Luvimer^{®} MAE or Luviflex^{®} Soft by the company BASF (INCI name: Acrylates copolymer), acrylic acid/butyl acrylate/methyl methacrylate terpolymers, such as the product sold under the name Balance^{®} CR by the company AkzoNobel (INCI name: Acrylates copolymer), or the copolymers of methacrylic acid and of methyl methacrylate sold under the name Eudragit^{®} L 100 by the company Rohm Pharma (INCI name: Acrylates copolymer). Mention may also be made of branched block polymers containing (meth)acrylic acid monomers, such as the product sold under the name Fixate^{®} G-100L by the company Lubrizol (INCI name: AMP-acrylates / allyl methacrylate copolymer);
B) crotonic acid copolymers, such as those including, in their chain, vinyl acetate or propionate units and optionally other monomers, such as allyl or methallyl esters, vinyl ether or vinyl ester of a saturated and linear or branched carboxylic acid bearing a long hydrocarbon-based chain, such as those including at least 5 carbon atoms, it being possible for these polymers optionally to be grafted or crosslinked, or alternatively another monomer which is a vinyl, allyl or methallyl ester of an α- or β-cyclic carboxylic acid. Such polymers are described, *inter alia,* in French patent Nos. 1 222 944, 1 580 545, 2 265 782, 2 265 781, 1 564 110 and 2 439 798. Commercial products which fall into this category are the products Resyn^{®} 28-2930 and 28-1310 sold by the company AkzoNobel (INCI names: VA / crotonates / vinyl decanoate copolymer and VA / crotonates copolymer, respectively). Mention may also be made of the products Luviset^{®} CA 66 sold by the company BASF, Aristoflex^{®} A60 sold by the company Clariant (INCI name: VA / crotonates copolymer) and Mexomere^{®} PW or PAM sold by the company Chimex (INCI name: VA / vinyl butyl benzoate / crotonates copolymer);
C) copolymers of C₄-C₈ monounsaturated carboxylic acids or anhydrides chosen from:
   - copolymers comprising (i) one or more maleic, fumaric or itaconic acids or anhydrides and (ii) at least one monomer chosen from vinyl esters, vinyl ethers, vinyl halides, phenylvinyl derivatives, acrylic acid and esters thereof, the anhydride functions of these copolymers optionally being monoesterified or monoamidated. Such polymers are described, in particular, in US patents 2 047 398, 2 723 248 and 2 102 113, and patent GB 839 805. Commercial products are in particular those sold under the names Gantrez^{®} AN or ES by the company ISP, such as Gantrez^{®} ES 225 (INCI name: Ethyl ester of PVM / MA copolymer) or Gantrez^{®} ES 425L (INCI name: Butyl ester of PVM / MA copolymer);
   - copolymers comprising (i) one or more maleic, citraconic or itaconic anhydride units and (ii) one or more monomers chosen from allyl or methallyl esters, optionally including one or more acrylamide, methacrylamide, α-olefin, acrylic or methacrylic ester, acrylic or methacrylic acid or vinylpyrrolidone groups in their chain,
      the anhydride functions of these copolymers optionally being monoesterified or monoamidated.
   These polymers are described, for example, in patents FR 2 350 384 and FR 2 357 241;
D) polyacrylamides including carboxylate groups.

The fixing polymers bearing units derived from sulfonic acid may be chosen from:
A') homopolymers and copolymers including vinylsulfonic, styrenesulfonic, naphthalenesulfonic or acrylamidoalkylsulfonic units.

These polymers may be notably chosen from:
- polyvinylsulfonic acid salts with a molecular mass of between approximately 1000 and 100 000, and also the copolymers with an unsaturated comonomer such as acrylic or methacrylic acids and esters thereof, and also acrylamide or derivatives thereof, vinyl ethers and vinylpyrrolidone;
- polystyrenesulfonic acid salts such as the sodium salts that are sold for example under the name Flexan^{®} II by Akzo Nobel (INCI name Sodium polystyrene sulfonate). These compounds are described in patent FR 2,198,719;
- polyacrylamidosulfonic acid salts, such as those mentioned in patent US 4 128 631, and more particularly the polyacrylamidoethylpropanesulfonic acid sold under the name Rheocare^{®} HSP-1180 by Cognis (INCI name polyacrylamidomethylpropane sulfonic acid);

B') sulfonic polyesters, these polymers being advantageously obtained by polycondensation of at least one dicarboxylic acid, of at least one diol or of a mixture of diol and of diamine, and of at least one difunctional monomer including a sulfonic function. Among these polymers, mention may be made of:
- linear sulfonic polyesters such as those described in patent applications US 3 734 874, US 3 779 993, US 4 119 680, US 4 300 580, US 4 973 656, US 5 660 816, US 5 662 893 and US 5 674 479. Such polymers are, for example, the products Eastman^{®} AQ38S Polymer, Eastman^{®} AQ55S Polymer and Eastman^{®} AQ48 Ultra Polymer sold by the company Eastman Chemical (name: Polyester-5) which are copolymers obtained from diethylene glycol, from 1,4-cyclohexanedimethanol, from isophthalic acid and from sulfoisophthalic acid salt.
- branched sulfonic polyesters such as those described in patent applications WO 95/18191, WO 97/08261 and WO 97/20899. Such compounds are, for example, the products Eastman^{®} AQ10D Polymer (name: Polyester-13) or Eastman^{®} AQ1350 Polymer provided by the company Eastman Chemical (name: Polyester-13).

According to the invention, the anionic fixing polymers are preferably chosen from copolymers of acrylic acid, such as the acrylic acid/ethyl acrylate/N-tert-butylacrylamide terpolymers sold notably under the name Ultrahold^{®} Strong by BASF, copolymers derived from crotonic acid, such as vinyl acetate/vinyl *tert-*butylbenzoate/crotonic acid terpolymers and the crotonic acid/vinyl acetate/vinyl neododecanoate terpolymers sold notably under the name Resyn 28-2930 by AkzoNobel, polymers derived from maleic, fumaric or itaconic acids or anhydrides with vinyl esters, vinyl ethers, vinyl halides, phenylvinyl derivatives and acrylic acid and esters thereof, such as the methyl vinyl ether/monoesterified maleic anhydride copolymers sold, for example, under the names Gantrez^{®} ES 425L or ES 225 by the company ISP, the copolymers of methacrylic acid and of ethyl acrylate sold under the name Luvimer^{®} MAE by the company BASF, and the vinyl acetate/crotonic acid copolymers sold under the name Luviset^{®} CA 66 by the company BASF, and the vinyl acetate/crotonic acid copolymers grafted with polyethylene glycol sold under the name Aristoflex^{®} A60 by the company Clariant, the vinylpyrrolidone/acrylic acid/lauryl methacrylate terpolymers sold under the name Acrylidone^{®} LM by the company ISP, the polymer sold under the name Fixate^{®} G-100L by the company Lubrizol, the vinyl acetate / crotonic acid/ vinyl p-tert-butylbenzoate copolymers sold under the names Mexomere^{®} PW or PAM by the company Chimex.

The cationic fixing polymers that may be used according to the present invention are preferably chosen from polymers including primary, secondary, tertiary and/or quaternary amine groups forming part of the polymer chain or directly attached thereto, and having a molecular weight of between 500 and approximately 5 000 000 and preferably between 1000 and 3 000 000.

Among these polymers, mention may be made more particularly of the following cationic polymers:
(1) homopolymers or copolymers derived from acrylic or methacrylic esters or amides and including at least one of the units of the following formulae: 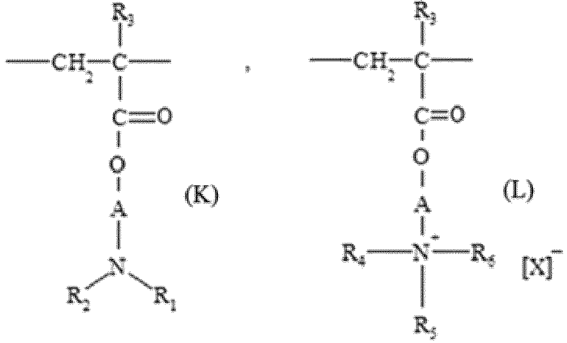 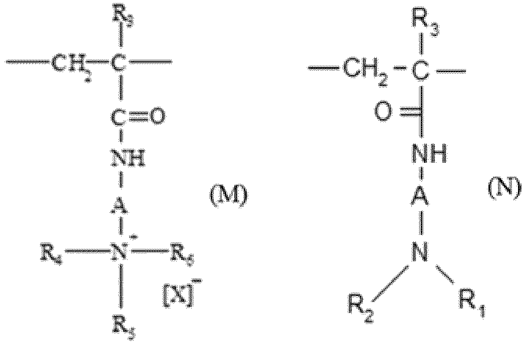 in which:
   - R₃ denotes a hydrogen atom or a CH₃ radical;
   - A is a linear or branched alkyl group including from 1 to 6 carbon atoms or a hydroxyalkyl group including from 1 to 4 carbon atoms;
   - R₄, R₅ and R₆, which may be identical or different, represent an alkyl group containing from 1 to 18 carbon atoms or a benzyl radical;
   - R₁ and R₂, which may be identical or different, each represent a hydrogen atom or an alkyl group containing from 1 to 6 carbon atoms;
   - X denotes a methosulfate anion or a halide such as chloride or bromide.
   The copolymers of class (1) also contain one or more units derived from comonomers which may be chosen from the class of acrylamides, methacrylamides, diacetone acrylamides, acrylamides and methacrylamides substituted on the nitrogen with lower (C₁-C₄) alkyl groups, groups derived from acrylic or methacrylic acids or esters thereof, vinyllactams such as vinylpyrrolidone or vinylcaprolactam, and vinyl esters.
   Thus, among these copolymers of class (1), mention may be made of:
   - quaternized or non-quaternized vinylpyrrolidone/dialkylaminoalkyl acrylate or methacrylate copolymers, such as the products sold under the name Gafquat^{®} by the company ISP, for instance Gafquat^{®} 734 or Gafquat^{®} 755 or Gafquat^{®} 755N (INCI name: Polyquaternium-11), or alternatively the products known as Copolymer^{®} 845, 958 and 937 sold by ISP (INCI name: VP/dimethylaminoethyl methacrylate copolymer). These polymers are described in detail in French patents 2 077 143 and 2 393 573,
   - polymers comprising a fatty chain and comprising a vinylpyrrolidone unit, such as the products sold under the names Styleze^{®} W20L and Styleze^{®} W10 by the company ISP (INCI name: Polyquaternium-55),
   - dimethylaminoethyl methacrylate/vinylcaprolactam/vinylpyrrolidone terpolymers, such as the products sold under the names Advantage HC 37 or Gaffix^{®} VC 713 by the company ISP (INCI name: Vinyl caprolactam / VP / dimethylaminoethyl methacrylate copolymer), and
   - quaternized vinylpyrrolidone/dimethylaminopropylmethacrylamide copolymers, such as the products sold under the name Gafquat^{®} HS 100 by the company ISP (name: Polyquaternium-28);
(2) cationic derivatives of guar gum, preferably comprising quaternary ammonium, such as those described in the United States patents 3 589 578 and 4 031 307, such as guar gums comprising cationic trialkylammonium groups. Such products are sold in particular under the trade names Jaguar^{®} C13 S, Jaguar^{®} C 15 and Jaguar^{®} C 17 by the company Rhodia (INCI name: Guar hydroxypropyltrimonium chloride);
(3) quaternary copolymers of vinylpyrrolidone and of vinylimidazole; mention may be made, for example, of vinylpyrrolidone / methyl vinylimidazolium chloride copolymers, such as the products sold by the company BASF under the names Luviquat^{®} FC550 or FC370, Luviquat^{®} Excellence, Luviquat^{®} Style (INCI name: Polyquaternium-16), or vinylpyrrolidone / vinylimidazolium methosulfate / vinylcaprolactam terpolymers, such as the product Luviquat^{®} Hold sold by the company BASF (INCI name: Polyquaternium-46);
(4) chitosans or salts thereof; the salts that may be used are in particular the acetate, lactate, glutamate, gluconate or pyrrolidonecarboxylate of chitosan.
   Among these compounds, mention may be made of the chitosan pyrrolidonecarboxylate sold under the name Kytamer^{®} PC by the company Amerchol (INCI name: Chitosan PCA);
(5) cationic cellulose derivatives such as copolymers of cellulose or of cellulose derivatives grafted with a water-soluble monomer including a quaternary ammonium, and described notably in patent US 4 131 576, such as hydroxyalkylcelluloses, for instance hydroxymethyl-, hydroxyethyl- or hydroxypropylcelluloses grafted notably with a methacryloyloxyethyltrimethylammonium, ethacrylamidopropyltrimethylammonium or dimethyldiallylammonium salt.

The products sold corresponding to this definition are more particularly the products sold under the names Celquat^{®} L 200 and Celquat^{®} H 100 by the company AkzoNobel (INCI name: Polyquaternium-4).

The amphoteric fixing polymers that may be used in accordance with the invention may be chosen from polymers including units L and M randomly distributed in the polymer chain, where L denotes a unit derived from a monomer including at least one basic nitrogen atom and M denotes a unit derived from an acidic monomer including one or more carboxylic or sulfonic groups or else L and M can denote groups derived from zwitterionic carboxybetaine or sulfobetaine monomers;

L and M may also denote a cationic polymer chain including primary, secondary, tertiary or quaternary amine groups, in which at least one of the amine groups bears a carboxylic or sulfonic group connected via a hydrocarbon-based group or alternatively L and M form part of a chain of a polymer bearing an ethylene-α,β-dicarboxylic unit in which one of the carboxylic groups has been made to react with a polyamine including one or more primary or secondary amine groups.

The amphoteric fixing polymers corresponding to the definition given above that are more particularly preferred are chosen from the following polymers:
(1) copolymers bearing acidic vinyl units and basic vinyl units, such as those resulting from the copolymerization of a monomer derived from a vinyl compound bearing a carboxylic group such as, more particularly, acrylic acid, methacrylic acid, maleic acid, α-chloroacrylic acid, and of a basic monomer derived from a substituted vinyl compound containing at least one basic atom, such as, more particularly, dialkylaminoalkyl methacrylate and acrylate, dialkylaminoalkylmethacrylamide and acrylamide. Such compounds are described in US patent 3 836 537;
(2) polymers including units derived:
   a) from at least one monomer chosen from acrylamides and methacrylamides substituted on the nitrogen atom with an alkyl group,
   b) from at least one acidic comonomer containing one or more reactive carboxylic groups, and
   c) from at least one basic comonomer such as esters with primary, secondary, tertiary and quaternary amine substituents of acrylic and methacrylic acids and the product of quaternization of dimethylaminoethyl methacrylate with dimethyl or diethyl sulfate.

The N-substituted acrylamides or methacrylamides that are more particularly preferred according to the invention are compounds in which the alkyl groups include from 2 to 12 carbon atoms and more particularly N-ethylacrylamide, N-tert-butylacrylamide, N-tert-octylacrylamide, N-octylacrylamide, N-decylacrylamide, N-dodecylacrylamide and the corresponding methacrylamides.

The acidic comonomers are more particularly chosen from acrylic acid, methacrylic acid, crotonic acid, itaconic acid, maleic acid and fumaric acid and alkyl monoesters, containing 1 to 4 carbon atoms, of maleic or fumaric acids or anhydrides.

The preferred basic comonomers are aminoethyl, butylaminoethyl, N,N'-dimethylaminoethyl and N-tert-butylaminoethyl methacrylates.

The copolymers whose INCI name is Octylacrylamide/acrylates/butylaminoethyl methacrylate copolymer, such as the products sold under the names Amphomer^{®}, Amphomer^{®} LV71 or Balance^{®} 47 by the company AkzoNobel, are particularly used;
(3) partially or totally acylated and crosslinked polyaminoamides derived from polyaminoamides of general formula: 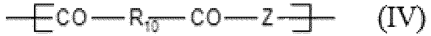 in which R₁₀ represents a divalent group derived from a saturated dicarboxylic acid, from an aliphatic mono- or dicarboxylic acid bearing an ethylenic double bond, from an ester of a lower alkanol containing from 1 to 6 carbon atoms of these acids, or from a group derived from the addition of any one of said acids to a bis-primary or bis-secondary amine, and Z denotes a group derived from a bis-primary, mono- or bis-secondary polyalkylenepolyamine and preferably represents:
a) in proportions of from 60 mol% to 100 mol%, the group 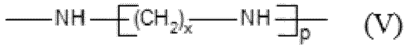 in which x = 2 and p = 2 or 3, or else x = 3 and p = 2,
   this group being derived from diethylenetriamine, from triethylenetetramine or from dipropylenetriamine;
b) in proportions of from 0 to 40 mol%, the group (V) above in which x = 2 and p = 1 and which is derived from ethylenediamine, or the group derived from piperazine: 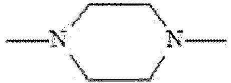
c) in proportions of from 0 to 20 mol%, the -NH-(CH₂)₆-NH- group being derived from hexamethylenediamine,

these polyaminoamides being crosslinked by addition reaction of a difunctional crosslinking agent chosen from epihalohydrins, diepoxides, dianhydrides and bis-unsaturated derivatives, using from 0.025 to 0.35 mol of crosslinking agent per amine group of the polyaminoamide and acylated by the action of acrylic acid, chloroacetic acid or an alkane sultone, or salts thereof.

The saturated carboxylic acids are preferably chosen from acids containing 6 to 10 carbon atoms, such as adipic acid, 2,2,4-trimethyladipic acid, 2,4,4-trimethyladipic acid and terephthalic acid, and acids bearing an ethylenic double bond, for instance acrylic, methacrylic and itaconic acids.

The alkane sultones used in the acylation are preferably propane sultone or butane sultone; the salts of the acylating agents are preferably the sodium or potassium salts;
(4) polymers including zwitterionic units of formula: 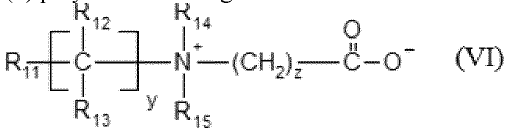 in which R₁₁ denotes a polymerizable unsaturated group, such as an acrylate, methacrylate, acrylamide or methacrylamide group, y and z represent an integer from 1 to 3, R₁₂ and R₁₃ represent a hydrogen atom or a methyl, ethyl or propyl group, and R₁₄ and R₁₅ represent a hydrogen atom or an alkyl group such that the sum of the carbon atoms in R₁₄ and R₁₅ does not exceed 10.

The polymers comprising such units may also include units derived from non-zwitterionic monomers such as dimethyl- or diethylaminoethyl acrylate or methacrylate or alkyl acrylates or methacrylates, acrylamides or methacrylamides or vinyl acetate.

Mention may be made, by way of example, of methyl methacrylate/methyl dimethylcarboxymethylammonioethyl methacrylate copolymers, such as the product sold under the name Diaformer Z-301N or Z-301W by the company Clariant (INCI name: Acrylates copolymer);
5) polymers derived from chitosan including monomer units corresponding to the following formulae: 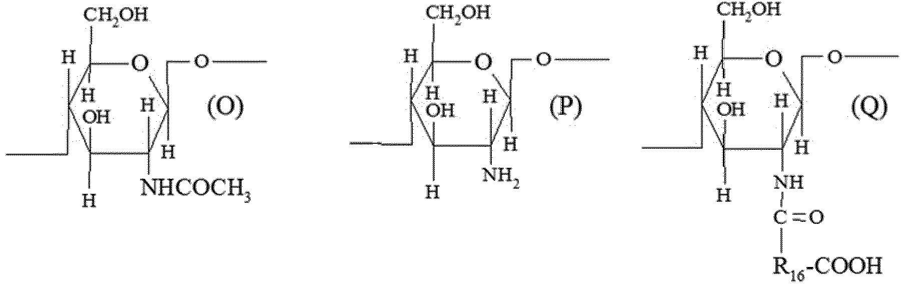 the unit (O) being present in proportions of between 0 and 30%, the unit (P) in proportions of between 5% and 50% and the unit (Q) in proportions of between 30% and 90%, it being understood that, in this unit (Q), R₁₆ represents a group of formula: 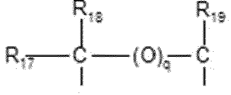
   in which, if q = 0, R₁₇, R₁₈ and R₁₉, which may be identical or different, each represent a hydrogen atom, a methyl, hydroxyl, acetoxy or amino residue, a monoalkylamine residue or a dialkylamine residue that are optionally interrupted with one or more nitrogen atoms and/or optionally substituted with one or more amine, hydroxyl, carboxyl, alkylthio or sulfonic groups, an alkylthio residue in which the alkyl group bears an amino residue, at least one of the groups R₁₇, R₁₈ and R₁₉ being, in this case, a hydrogen atom;
   or, if q = 1, R₁₇, R₁₈ and R₁₉ each represent a hydrogen atom, and also the salts formed by these compounds with bases or acids;
(6) polymers containing units corresponding to general formula (VII) are described, for example, in French patent 1 400 366: 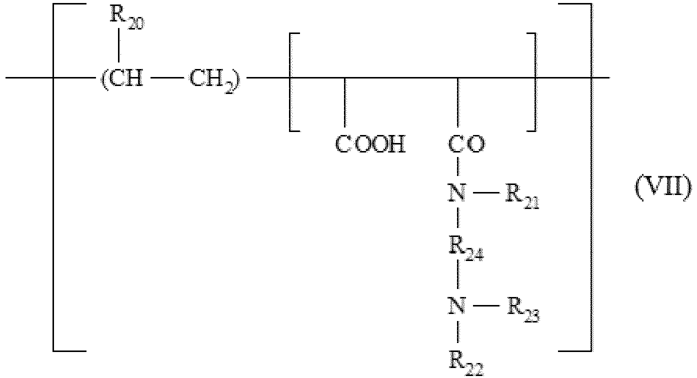 in which R₂₀ represents a hydrogen atom, a CH₃O, CH₃CH₂O or phenyl group, R₂₁ denotes a hydrogen atom or a lower alkyl group such as methyl or ethyl, R₂₂ denotes a hydrogen atom or a C₁₋₆ lower alkyl group such as methyl or ethyl, R₂₃ denotes a C₁₋₆ lower alkyl group such as methyl or ethyl or a group corresponding to the formula: -R₂₄-N(R₂₂)₂, with R₂₄ representing a -CH₂-CH₂-, -CH₂-CH₂-CH₂-, or - CH₂-CH(CH₃)- group and R₂₂ having the meanings given above;
(7) polymers derived from the N-carboxyalkylation of chitosan, such as N-carboxymethyl chitosan or N-carboxybutyl chitosan, for instance the product sold under the name Chitoglycan by the company Sinerga SPA (INCI name: Carboxymethyl chitosan);
(8) amphoteric polymers of the -D-X-D-X type chosen from:
   a) polymers obtained by the action of chloroacetic acid or sodium chloroacetate on compounds including at least one unit of formula:

      -D-X-D-X-D- (VIII)

      in which D denotes a group 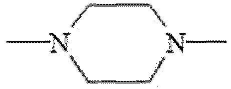 and X denotes the symbol E or E', where E and E', which may be identical or different, denote a divalent group that is an alkylene group with a straight or branched chain including up to 7 carbon atoms in the main chain, which is unsubstituted or substituted with hydroxyl groups and which may include, in addition to oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups;
   b) polymers of formula:

      -D-X-D-X- (VIII')

      in which D denotes a group 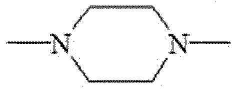 and X denotes the symbol E or E' and at least once E'; E having the meaning given above and E' being a divalent group that is an alkylene group with a straight or branched chain containing up to 7 carbon atoms in the main chain, which is unsubstituted or substituted with one or more hydroxyl groups and which includes one or more nitrogen atoms, the nitrogen atom being substituted with an alkyl chain that is optionally interrupted with an oxygen atom and which necessarily includes one or more carboxyl functions or one or more hydroxyl functions betainized by reaction with chloroacetic acid or sodium chloroacetate;
(9) (C₁-C₅)alkyl vinyl ether/maleic anhydride copolymers partially modified by semiamidation with an N,N-dialkylaminoalkylamine, such as N,N-dimethylaminopropylamine, or by semiesterification with an N,N-dialkylaminoalkanol. These copolymers may also include other vinyl comonomers, such as vinylcaprolactam.

Among the amphoteric fixing polymers mentioned above that are most particularly preferred according to the invention, mention will be made of those of family (3), such as the copolymers whose INCI name is Octylacrylamide/acrylates/butylaminoethyl methacrylate copolymer, such as the products sold under the names Amphomer^{®}, Amphomer^{®} LV 71 or Balance^{®} 47 by the company AkzoNobel and those of family (4), such as the methyl methacrylate/methyl dimethylcarboxymethylammonioethyl methacrylate copolymers sold, for example, under the name Diaformer Z-301N or Z-301W by the company Clariant.

The nonionic fixing polymers that may be used according to the present invention are chosen, for example, from:
- polyalkyloxazolines;
- vinyl acetate homopolymers,
- vinyl acetate copolymers, for instance copolymers of vinyl acetate and of acrylic ester, copolymers of vinyl acetate and of ethylene, or copolymers of vinyl acetate and of maleic ester, for example of dibutyl maleate;
- homopolymers and copolymers of acrylic esters, for instance copolymers of alkyl acrylates and of alkyl methacrylates, such as the products provided by the company Röhm GmbH under the name Eudragit^{®} NE 30 D (INCI name: Acrylates copolymer);
- copolymers of acrylonitrile and of a nonionic monomer chosen, for example, from butadiene and alkyl (meth)acrylates;
- styrene homopolymers;
- styrene copolymers, for instance copolymers of styrene, of alkyl acrylate and of alkyl methacrylate; copolymers of styrene and of butadiene; or copolymers of styrene, of butadiene and of vinylpyridine;
- polyamides;
- vinyllactam homopolymers, such as the vinylpyrrolidone homopolymers sold, for example, under the names Luviskol^{®} K30 powder by the company BASF or PVP K30L or K60 solution or K90 by the company ISP, or such as the polyvinylcaprolactam sold under the name Luviskol^{®} Plus by the company BASF (INCI name: PVP);
- vinyllactam copolymers, such as a poly(vinylpyrrolidone/vinyllactam) copolymer sold under the trade name Luvitec^{®} VPC 55K65W by the company BASF, poly(vinylpyrrolidone/vinyl acetate) copolymers, such as those sold under the name PVP/VA^{®} S630L, E735, E635 and W735 by the company ISP, Luviskol^{®} VA 73, VA 64 and VA 37 by the company BASF (INCI name: VP/VA copolymer); and vinylpyrrolidone/methacrylamide/vinylimidazole terpolymers, for instance the product sold under the name Luviset^{®} Clear by the company BASF (INCI name: VP/methacrylamide/vinyl imidazole copolymer).

The alkyl groups of the nonionic polymers mentioned above preferably contain from one to six carbon atoms.

Use may also be made, according to the invention, of fixing polymers of grafted silicone type comprising a polysiloxane portion and a portion constituted of a non-silicone organic chain, one of the two portions constituting the main chain of the polymer and the other being grafted onto said main chain.

These polymers are described, for example, in patent applications EP-A-0 412 704, EP-A-0 412 707, EP-A-0 640 105 and WO 95/00578, EP-A-0 582 152 and WO 93/23009, and patents US 4 693 935, US 4 728 571 and US 4 972 037.

These polymers may be amphoteric, anionic or nonionic and they are preferably anionic or nonionic.

Such polymers are, for example, copolymers that may be obtained by free radical polymerization from the monomer mixture formed from:
a) 50% to 90% by weight of *tert*-butyl acrylate,
b) 0 to 40% by weight of acrylic acid,
c) 5% to 40% by weight of a silicone macromer of formula: 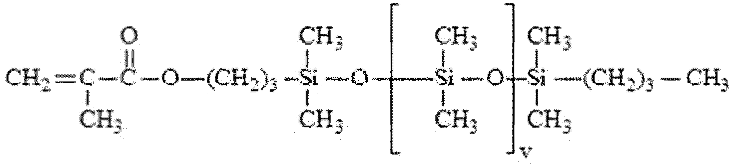 in which v is a number ranging from 5 to 700, the weight percentages being calculated relative to the total weight of the monomers.

Other examples of grafted silicone polymers are notably polydimethylsiloxanes (PDMSs) to which are grafted mixed polymer units of the poly((meth)acrylic acid) type and of the poly(alkyl (meth)acrylate) type via a thiopropylene-type connecting link and polydimethylsiloxanes (PDMSs) to which polymer units of the poly(isobutyl (meth)acrylate) type are grafted via a thiopropylene-type connecting link.

Grafted silicone polymers are sold, for example, under the names Silicone Plus Polymer^{®} VS80 and VA70 by 3M (INCI names: Polysilicone-8 and Polysilicone-7, respectively).

Another type of silicone fixing polymer that may be mentioned is the product Luviflex^{®} Silk sold by the company BASF (INCI name: PEG/PPG-25/25 dimethicone/acrylates copolymer).

Functionalized or non-functionalized, silicone or non-silicone, cationic, nonionic, anionic or amphoteric polyurethanes or mixtures thereof may also be used as fixing polymers.

The polyurethanes particularly targeted by the present invention are those described in patent applications EP 0 751 162, EP 0 637 600, EP 0 648 485 and FR 2 743 297, of which the applicant is the proprietor, and also in patent applications EP 0 656 021 and WO 94/03510 from the company BASF and EP 0 619 111 from the company National Starch.

As polyurethanes that are particularly suitable for use in the present invention, mention may be made of the products sold under the names Luviset PUR^{®} and Luviset^{®} Si PUR by the company BASF (INCI names: Polyurethane-1 and Polyurethane-6, respectively).

When the composition comprises one or more fixing polymers, their total content preferably ranges from 0.05% to 10% by weight and preferentially from 0.1% to 5% by weight relative to the total weight of the composition.

### Powders

The cosmetic composition contained in the aerosol device according to the present invention may optionally also comprise one or more powders.

The powders may be chosen from fillers, pigments, and mixtures thereof.

Preferably, the composition according to the invention may comprise one or more styling powders.

For the purposes of the present invention, the term "styling powder" means a powder which has a capacity for shaping the head of hair or for the durability of this shaping.

The capacity for shaping or ensuring shaping durability of the powder may notably be due to its chemical nature and/or its geometrical form and/or its arrangement configuration during deposition onto the keratin fibre. Specifically, the irregularities created at the surface of the hair promote the attachment of the fibres to each other.

The powder may be of any form, such as lamellar, spherical or oblong, irrespective of the crystallographic form (for example cubic, hexagonal, orthorhombic, rhombohedric or tetragonal). In a preferred embodiment, the powders are not spherical.

The number-average size of the powder may range from 0.001 to 50 µm, better still from 0.002 to 40 µm and even more preferentially from 0.003 to 35 µm.

This number-average size corresponds to the size measured from the statistical distribution of the particle sizes for half of the total number of the particles. This size is referred to as the D50.

In addition, the number-average size of these particles may be measured in the form of a mean value via an observation method with a light microscope, an electronic microscope, or a particle size analyser using laser scattering.

In the case where the particles are not in spherical form, their number-average size may be determined in the form of a mean of the longest or shortest diameter or of the thickness.

Said powder may be mineral or organic, preferably mineral.

The powder is preferably chosen from pigments and fillers, and mixtures thereof.

For the purposes of the present invention, the term "filler" means natural or synthetic, white or colourless particles of any form, which are insoluble in the medium of the composition, irrespective of the temperature at which the composition is manufactured.

The fillers may be organic or inorganic, and may be of any form such as lamellar, spherical or oblong, irrespective of the crystallographic form (for example cubic, hexagonal, orthorhombic, rhombohedric or tetragonal). In a preferred embodiment, the fillers are not spherical.

For the purposes of the present invention, the term "pigment" means organic or mineral, white or coloured particles of any form, which are insoluble in physiological medium and which give the composition a colour.

The term "mineral" encompasses natural or synthetic chemical compounds that are inorganic. Mineral substances are mainly in a crystalline form.

Examples of mineral or inorganic powders that may notably be mentioned include:
- fillers such as metal carbonates, oxides and sulfates such as those of alkaline-earth metals, aluminium, gallium and indium; silicates; modified or unmodified silicas; sericite, synthetic fluorophlogopite, talc; natural or synthetic mica, notably white mica, gold mica, red mica, black mica and mica-lithium oxide; calcium phosphate, silicic acid, silicic anhydride, silicon carbide, metal salts of tungstic acid, magnesium aluminate, bentonite, zeolites, smectite, hydroxyapatite, ceramic powder, boron nitride and glass or ceramic microcapsules;
- specific composite fillers such as those sold under the names Excel Mica, Excel Pearl and the powder La Vie by the company Miyoshi Kasei, Inc.;
- white pigments such as titanium dioxide, zinc oxide, zirconium oxide and cerium oxide;
- coloured pigments such as red iron oxide, yellow iron oxide, black iron oxide, chromium oxide, chromium hydroxide, Prussian blue, ultramarine blue, chromium hydrate, ferric blue, inorganic blue pigments, carbon black, lower titanium oxides, manganese violet, cobalt violet, and metal powders such as aluminium powder and copper powder;
- nacreous pigments such as bismuth oxychloride, mica/titanium, essence of pearl, powder prepared by coating synthetic mica with titanium dioxide, powder prepared by coating silica flakes with titanium dioxide, which is available under the brand name Metashine from Nippon Sheet Glass Co., Ltd, powder prepared by coating alumina flakes with tin oxide and titanium dioxide, powder prepared by coating aluminium flakes with titanium dioxide, powder prepared by coating copper flakes with silica, sold by the company Eckert Inc. USA, powder prepared by coating bronze flakes with silica, and powder prepared by coating aluminium flakes with silica;
- ultrafine powder, having a mean particle size of less than 0.1 µm, such as ultrafine titanium dioxide, ultrafine zinc oxide, ultrafine iron oxide, and ultrafine cerium oxide;
- other powders such as the luminescent powder sold under the brand name Luminova Series by Mitsui & Co., Ltd., aluminium powder, stainless-steel powder, tourmaline powder, and amber powder; and
- a mixture thereof.

Examples of organic powders are wool powder, polyamide powder (Nylon^{®} or Orgasol^{®} from Arkema), polyester powder, polyethylene powder, polypropylene powder, polystyrene powder, polyurethane powder, benzoguanamine powder, polymethyl benzoguanamine powder, tetrafluoroethylene powder, poly(methyl methacrylate) powder, cellulose powder, silk powder, silicone powder, silicone rubber powder, powders of synthetic resins such as a styrene/acrylate copolymer, a divinylbenzene/styrene copolymer, a vinyl resin, a urea resin, a phenolic resin, a fluoro resin, tetrafluoroethylene (Teflon^{®}) polymers, a silicone resin, an acrylic resin, a melamine resin, an epoxy resin, and a polycarbonate resin, hollow polymer microspheres, such as those of poly(vinylidene chloride)/acrylonitrile, for example Expancel^{®} (Nobel Industrie), or acrylic acid copolymers (Polytrap^{®} from the company Dow Corning), silicone resin microbeads (for example Tospearls^{®} from Toshiba), particles formed from polyorganosiloxane elastomers, microcrystalline fibre powder, starch powder, acylated lysine powder, poly-β-alanine, lauryllysine, powder of the metal salt of (long-chain alkyl) phosphate, metal soap powder, Colour Index (CI) yellow pigments, CI orange pigments and tar-based pigments prepared in lacquer form, and dyes existing in the natural state prepared in lacquer form.

The doped or undoped composite powder may also be suitable as base powder intended to undergo a surface treatment. Examples of the latter include the powder prepared by coating inorganic dye pigments such as red iron oxide with silicic anhydride, powders prepared by coating Nylon with white pigments and powders prepared by coating fillers with ultrafine white pigments.

The mineral powder(s) according to the invention may optionally be surface-modified with organic compounds.

More preferentially, the powder is a mineral powder constituted of one or more water-insoluble mineral compounds.

For the purposes of the present invention, the term "water-insoluble" refers to a compound whose solubility at spontaneous pH in water at 25°C and at atmospheric pressure is less than 0.1%.

The water-insoluble mineral compound(s) are preferably chosen from metal carbonates, oxides and sulfates, silicates, modified or unmodified silicas, mica, talc, and mixtures thereof.

Examples that may more particularly be mentioned include the carbonates, oxides and sulfates of alkaline-earth metals such as beryllium, magnesium, calcium, strontium, barium and radium, better still magnesium and calcium; the oxides, sulfates and carbonates of aluminium, gallium and indium; silicates such as kaolinite or kaolins (natural silicates containing kaolinite), silicates containing magnesium, particularly those containing an amount of magnesium of greater than 10% by weight (on a dry basis) expressed as magnesium oxide, such as Li-Mg-Na silicates, for instance Laponite XLG, provided by the company Rockwood; modified or unmodified silicas, better still modified silicas; mica; talc; and mixtures thereof.

Among the modified silicas, it is preferred to use surface-treated silicas such as hydrophobic silicas, for instance hydrophobic fumed silica of nanometric size and surface-treated with hexamethyldisilazane, such as the silica sold under the trade name Aerosil R812S or Aerosil R972 by the company Evonik, or HDK H115 by the company Wacker, or the hydrophobic fumed silica surface-treated with dimethylsilane.

More preferentially, the powder(s) are chosen from fillers, and more preferentially from calcium carbonate, magnesium carbonate, alumina, barium sulfate, magnesium oxide, kaolinite or kaolins, modified or unmodified silicas, better still modified silicas and even better still hydrophobic fumed silica of nanometric size and surface-treated with hexamethyldisilazane or hydrophobic fumed silica surface-treated with dimethylsilane; mica; talc; and mixtures thereof.

When the composition comprises one or more powders, their total content preferably ranges from 0.05% to 10% by weight and preferentially from 0.1% to 5% by weight relative to the total weight of the composition.

### Additives

The cosmetic composition contained in the aerosol device according to the present invention may also optionally comprise one or more additives, other than the compounds of the invention and among which mention may be made of cationic, nonionic, anionic, amphoteric or zwitterionic surfactants, and mixtures thereof, cationic, anionic, nonionic or amphoteric polymers, or mixtures thereof, other than the fixing polymers of the invention, antidandruff agents, anti-seborrhoea agents, vitamins and provitamins including panthenol, sunscreens, sequestrants, plasticizers, solubilizers, acidifying agents, alkaline agents, mineral or organic thickeners, notably polymeric thickeners, antioxidants, hydroxy acids, fragrances and preserving agents.

Needless to say, a person skilled in the art will take care to select this or these optional additional compound(s) such that the advantageous properties intrinsically associated with the composition according to the invention are not, or are not substantially, adversely affected by the envisaged addition(s).

The above additives may generally be present in an amount, for each of them, of between 0 and 20% by weight relative to the total weight of the composition.

The aerosol device comprising the cosmetic composition according to the present invention consists of a container containing said composition, and of a means for spraying said composition.

Preferably, the aerosol device according to the invention makes it possible to spray said cosmetic composition in foam form.

The cosmetic composition according to the invention is advantageously packaged under pressure, in an aerosol device, for example a monobloc device, which comprises a spraying means and a container.

The spraying means is generally formed from a dispensing valve controlled by a dispensing head, which itself comprises a nozzle via which the composition of the invention is sprayed, preferably in foam form.

The container containing the pressurized composition may be opaque or transparent. It may be made of glass, polymer or metal, and may optionally be coated with a protective varnish coat.

A subject of the invention is also a cosmetic process for treating keratin fibres, in particular human keratin fibres, in particular human keratin fibres such as the hair, comprising a step of applying to said keratin fibres a cosmetic composition sprayed from an aerosol device as defined previously.

The cosmetic composition according to the invention may be applied to dry or damp keratin materials that have optionally been washed with shampoo.

A subject of the invention is also the use of a cosmetic composition sprayed from an aerosol device according to the present invention, for conditioning and/or shaping keratin fibres, in particular human keratin fibres such as the hair.

In a particular embodiment of the invention, the composition according to the invention is used for shaping keratin materials, in particular human keratin materials such as the hair, notably for curl relaxing and curl definition.

The examples that follow serve to illustrate the invention without, however, being limiting in nature.

### Examples

In the examples that follow, all the amounts are indicated as weight percentages of active material relative to the total weight of the composition.

### Example 1

### a) Test composition

A composition was prepared from the ingredients whose contents are indicated in the table below:

**[Table 1]**

| | Composition |
|---|---|
| Stearoylsarcosine | 0.29 |
| Cetyl alcohol | 1.96 |
| Myristoylsarcosine | 0.1 |
| Triethanolamine | 0.18 |
| Preserving agent, fragrance | qs |
| Water | qs 100 |
| Propellant | 2 |

### b) Packaging

The composition thus obtained was packaged in two 150 mL containers A and B with a valve and a foam-type diffuser.

The propellant contained in the first container A is an isobutane/butane/propane mixture (comparative), whereas the propellant contained in the second container B is carbon dioxide (according to the invention).

The foams obtained after spraying these compositions are evaluated visually.

### c) Results

The composition sprayed using the device according to the invention (B) has better foam properties than that sprayed with the comparative device (A).

Specifically, the foam obtained using the composition packaged with carbon dioxide (according to the invention) is creamy. It also has a shiny appearance and also a creamy and well-formed texture, which is easy to apply.

Conversely, the foam obtained using the composition packaged with a propellant of hydrocarbon type (comparative) is expanded and very airy. It also has a matt appearance and a light, volatile texture. It is thus more difficult to apply and less pleasant than the foam obtained with the device of the invention.

### Example 2

### a) Test compositions

Composition B1 according to the present invention and comparative composition A1 were prepared using the ingredients whose contents are indicated in the table below:

**[Table 2]**

| | A1 (comparative) | B1 (invention) |
|---|---|---|
| Stearoylsarcosine | 0.29 | 0.29 |
| **Cetyl alcohol** | - | **1.96** |
| Myristoylsarcosine | 0.1 | 0.1 |
| Triethanolamine | 0.18 | 0.18 |
| Preserving agent, fragrance | qs | qs |
| Water | qs 100 | qs 100 |
| Carbon dioxide | 2 | 2 |

### b) Packaging

The compositions are packaged in a 150 mL container with a valve and a foam-type diffuser.

The foams obtained after spraying these compositions are evaluated visually.

### b) Results

The composition according to the invention (comprising a fatty alcohol) has better foam properties than the comparative composition (not comprising any fatty alcohol).

Specifically, the foam obtained using composition B1 is creamy with a glossy appearance and a creamy and well-formed texture, which is easy to apply.

Conversely, the foam obtained using composition A1 has an unstable liquid texture. It is thus more difficult to apply to the hair.

### Example 3

### a) Test compositions

Composition B2 according to the present invention and comparative composition A2 were prepared using the ingredients whose contents are indicated in the table below.

The weight ratio R between the content of fatty acid(s) and the content of anionic surfactant(s) was calculated for each composition.

**[Table 3]**

| | A2 (comparative) | B2 (invention) |
|---|---|---|
| Cetyl alcohol | 1.96 | 1.96 |
| Stearic acid | 1.96 | 0.39 |
| Triethanolamine | 0.98 | 0.18 |
| Preserving agent, fragrance | qs | qs |
| Water | qs 100 | qs 100 |
| Carbon dioxide | 2 | 2 |
| **R** | **1** | **5** |

### b) Packaging

The compositions are packaged in a 150 mL container with a valve and a foam-type diffuser.

The foams obtained after spraying these compositions are evaluated visually.

### b) Results

The composition according to the invention (with R ≥ 1.5) has better foam properties than the comparative composition (with R < 1.5).

Specifically, the foam obtained using composition B2 (R = 5) is creamy with a glossy appearance and a creamy and well-formed texture, which is easy to apply.

Conversely, the foam obtained using composition A2 (R = 1) has a matt, nonhomogeneous appearance with the presence of nacreous crystals and an unstable texture. The foam thus obtained is difficult to apply to the hair.

## Claims

1. Aerosol device containing a cosmetic composition comprising:
- one or more anionic surfactants,
- one or more fatty alcohols, and
- one or more propellants chosen from compressed gases,
the weight ratio between the total content of the fatty alcohol(s) and the total content of the anionic surfactant(s) ranging from 1.5 to 10.

2. Aerosol device according to Claim 1, **characterized in that** the anionic surfactant(s) are chosen from carboxylate anionic surfactants, and mixtures thereof; preferably from C₆-C₃₀ fatty acids, acyl(C₆-C₃₀)glycinates, acyl(C₆-C₃₀)lactylates, acyl(C₆-C₃₀)sarcosinates, acyl(C₆-C₃₀)glutamates; alkyl-D-galactosiduronic acids, alkyl ether carboxylic acids, alkyl(C₆-C₃₀ aryl) ether carboxylic acids, alkylamido ether carboxylic acids; monoesters of C₆-C₂₄ alkyl and of polyglycoside-polycarboxylic acids; and also the salts of these compounds; and mixtures thereof; more preferably from (C₆-C₂₄)acyl sarcosinates, C₆-C₂₄ fatty acids and mixtures thereof; even preferably from stearoylsarcosine, myristoylsarcosine, myristic acid, stearic acid, palmitic acid, salts thereof and mixtures thereof.

3. Device according to any one of the preceding claims, **characterized in that** the total content of anionic surfactant(s) is greater than or equal to 0.05% by weight, preferably greater than or equal to 0.1% by weight, preferentially ranges from 0.1% to 5% by weight and more preferentially from 0.2% to 1% by weight, relative to the total weight of the composition.

4. Device according to any one of the preceding claims, **characterized in that** the fatty alcohol(s) are chosen from solid fatty alcohols, and mixtures thereof, preferably from saturated or unsaturated, linear or branched solid fatty alcohols including from 8 to 40 carbon atoms, better still from saturated linear solid fatty alcohols, and mixtures thereof, even better still from cetyl alcohol, stearyl alcohol, behenyl alcohol, myristyl alcohol, and mixtures thereof.

5. Device according to any one of the preceding claims, **characterized in that** the total content of the fatty alcohol(s) ranges from 0.05% to 10% by weight, preferably from 0.1% to 8% by weight and more preferentially from 1% to 5% by weight, relative to the total weight of the composition.

6. Device according to any one of the preceding claims, **characterized in that** the weight ratio between the total content of the fatty alcohol(s) and the total content of the anionic surfactant(s) ranges from 2 to 8, preferably from 3 to 7 and even more preferentially from 4 to 6.

7. Device according to any one of the preceding claims, **characterized in that** the weight ratio between the total content of solid fatty alcohol(s) and the total content of carboxylate anionic surfactants ranges from 1.5 to 10, preferably from 2 to 8, more preferentially from 3 to 7 and even more preferentially from 4 to 6.

8. Device according to any one of the preceding claims, **characterized in that** the propellant(s) are chosen from air, nitrogen, carbon dioxide, and mixtures thereof, preferably carbon dioxide, and preferably the total content of said propellant(s) ranges from 0.5% to 10% by weight, preferably from 1% to 5% by weight and more preferentially from 1.5% to 4% by weight, relative to the total weight of the composition.

9. Device according to any one of the preceding claims, **characterized in that** the cosmetic composition also comprises one or more fatty substances other than fatty alcohols, preferably one or more liquid fatty substances other than fatty alcohols, preferably in a total content ranging from 0.05% to 10% by weight, preferentially from 0.1% to 5% by weight relative to the total weight of the composition.

10. Device according to any one of the preceding claims, **characterized in that** the cosmetic composition also comprises one or more fixing polymers, preferably in a total content ranging from 0.05% to 10% by weight, preferentially from 0.1% to 5% by weight relative to the total weight of the composition.

11. Device according to any one of the preceding claims, **characterized in that** the cosmetic composition also comprises one or more powders, preferably in a total content ranging from 0.05% to 10% by weight, preferentially from 0.1% to 5% by weight relative to the total weight of the composition.

12. Device according to any one of the preceding claims, **characterized in that** the cosmetic composition also comprises water, preferably in a content preferably ranging from 10% to 99% by weight, preferentially from 30% to 98% by weight, better still from 50% to 97% by weight, better still from 80% to 96% by weight relative to the total weight of the composition.

13. Cosmetic process for treating keratin fibres, in particular human keratin fibres such as the hair, comprising a step of applying to said keratin fibres a cosmetic composition sprayed from an aerosol device as defined in any one of the preceding claims.

14. Use of a cosmetic composition sprayed from an aerosol device according to any one of Claims 1 to 12, for conditioning and/or shaping keratin fibres, in particular human keratin fibres such as the hair.

## Patentansprüche

1. Aerosolvorrichtung, die eine kosmetische Zusammensetzung enthält, umfassend:
- ein oder mehrere anionische Tenside,
- ein oder mehrere Fettalkohole, und
- ein oder mehrere Treibmittel ausgewählt aus komprimierten Gasen,
wobei das Gewichtsverhältnis zwischen dem Gesamtgehalt des oder der Fettalkohole und dem Gesamtgehalt des oder der anionischen Tenside im Bereich von 1,5 bis 10 liegt.

2. Aerosolvorrichtung nach Anspruch 1, **dadurch gekennzeichnet, dass** das oder die anionischen Tenside ausgewählt ist/sind aus anionischen Carboxylattensiden und Mischungen davon; vorzugsweise aus C₆-C₃₀-Fettsäuren, Acyl(C₆-C₃₀)glycinaten, Acyl(C₆-C₃₀)lactylaten, Acyl (C₆-C₃₀)sarcosinaten, Acyl (C₆-C₃₀)glutamaten; Alkyl-D-galactosiduronsäuren, Alkylethercarbonsäuren, Alkyl (C₆-C₃₀-aryl)ethercarbonsäuren, Alkylamidoethercarbonsäuren; Monoestern von C₆-C₂₄-Alkyl- und von Polyglycosidpolycarbonsäuren; sowie den Salzen dieser Verbindungen; und Mischungen davon; besonders bevorzugt von (C₆-C₂₄)Acylsarcosinaten, C₆-C₂₄-Fettsäuren und Mischungen davon; sogar vorzugsweise von Stearoylsarcosin, Myristoylsarcosin, Myristinsäure, Stearinsäure, Palmitinsäure, Salzen davon und Mischungen davon.

3. Vorrichtung nach einem der vorhergehenden Ansprüche, **dadurch gekennzeichnet, dass** der Gesamtgehalt an anionischem Tensid oder an anionischen Tensiden größer oder gleich 0,05 Gew.-%, vorzugsweise größer oder gleich 0,1 Gew.-%, vorzugsweise im Bereich von 0,1 bis 5 Gew.-% und bevorzugter im Bereich von 0,2 bis 1 Gew.-% liegt, bezogen auf das Gesamtgewicht der Zusammensetzung.

4. Vorrichtung nach einem der vorhergehenden Ansprüche, **dadurch gekennzeichnet, dass** der oder die Fettalkohole ausgewählt ist/sind aus festen Fettalkoholen und Mischungen davon, vorzugsweise aus gesättigten oder ungesättigten, linearen oder verzweigten festen Fettalkoholen mit 8 bis 40 Kohlenstoffatomen, besser noch aus gesättigten linearen festen Fettalkoholen und Mischungen davon, noch besser aus Cetylalkohol, Stearylalkohol, Behenylalkohol, Myristylalkohol und Mischungen davon.

5. Vorrichtung nach einem der vorhergehenden Ansprüche, **dadurch gekennzeichnet, dass** der Gesamtgehalt des oder der Fettalkohole im Bereich von 0,05 bis 10 Gew.-%, vorzugsweise von 0,1 bis 8 Gew.-% und bevorzugter von 1 bis 5 Gew.-% liegt, bezogen auf das Gesamtgewicht der Zusammensetzung.

6. Vorrichtung nach einem der vorhergehenden Ansprüche, **dadurch gekennzeichnet, dass** das Gewichtsverhältnis zwischen dem Gesamtgehalt des oder der Fettalkohole und dem Gesamtgehalt des oder der anionischen Tenside im Bereich von 2 bis 8, vorzugsweise 3 bis 7 und noch bevorzugter von 4 bis 6 liegt.

7. Vorrichtung nach einem der vorhergehenden Ansprüche, **dadurch gekennzeichnet, dass** das Gewichtsverhältnis zwischen dem Gesamtgehalt an festem Fettalkohol oder festen Fettalkoholen und dem Gesamtgehalt an anionischen Carboxylattensiden im Bereich von 1,5 bis 10, vorzugsweise von 2 bis 8, bevorzugter von 3 bis 7 und noch bevorzugter von 4 bis 6 liegt.

8. Vorrichtung nach einem der vorhergehenden Ansprüche, **dadurch gekennzeichnet, dass** das oder die Treibmittel ausgewählt ist/sind aus Luft, Stickstoff, Kohlendioxid und Mischungen davon, vorzugsweise Kohlendioxid, und vorzugsweise der Gesamtgehalt des Treibmittels oder der Treibmittel im Bereich von 0,5 bis 10 Gew.-%, vorzugsweise von 1 bis 5 Gew.-% und bevorzugter von 1,5 bis 4 Gew.-% liegt, bezogen auf das Gesamtgewicht der Zusammensetzung.

9. Vorrichtung nach einem der vorhergehenden Ansprüche, **dadurch gekennzeichnet, dass** die kosmetische Zusammensetzung auch eine oder mehrere Fettsubstanzen umfasst, die von Fettalkoholen verschieden ist/sind, vorzugsweise eine oder mehrere flüssige Fettsubstanzen, die von Fettalkoholen verschieden ist/sind, vorzugsweise in einem Gesamtgehalt im Bereich von 0,05 bis 10 Gew.-%, vorzugsweise von 0,1 bis 5 Gew.-%, bezogen auf das Gesamtgewicht der Zusammensetzung.

10. Vorrichtung nach einem der vorhergehenden Ansprüche, **dadurch gekennzeichnet, dass** die kosmetische Zusammensetzung auch ein oder mehrere Fixierungspolymere umfasst, vorzugsweise in einem Gesamtgehalt im Bereich von 0,05 bis 10 Gew.-%, vorzugsweise von 0,1 bis 5 Gew.-%, bezogen auf das Gesamtgewicht der Zusammensetzung.

11. Vorrichtung nach einem der vorhergehenden Ansprüche, **dadurch gekennzeichnet, dass** die kosmetische Zusammensetzung auch ein oder mehrere Pulver umfasst, vorzugsweise in einem Gesamtgehalt im Bereich von 0,05 bis 10 Gew.-%, vorzugsweise von 0,1 bis 5 Gew.-%, bezogen auf das Gesamtgewicht der Zusammensetzung.

12. Vorrichtung nach einem der vorhergehenden Ansprüche, **dadurch gekennzeichnet, dass** die kosmetische Zusammensetzung auch Wasser umfasst, vorzugsweise in einem Gehalt vorzugsweise im Bereich von 10 bis 99 Gew.-%, vorzugsweise von 30 bis 98 Gew.-%, bevorzugter von 50 bis 97 Gew.-% und noch besser von 80 bis 96 Gew.-%, bezogen auf das Gesamtgewicht der Zusammensetzung.

13. Kosmetisches Verfahren zur Behandlung von Keratinfasern, insbesondere menschlichen Keratinfasern, wie dem Haar, umfassend einen Schritt des Aufbringens einer kosmetischen Zusammensetzung, die aus einer Aerosolvorrichtung gemäß einem der vorhergehenden Ansprüche gesprüht wird, auf die Keratinfasern.

14. Verwendung einer kosmetischen Zusammensetzung, die aus einer Aerosolvorrichtung gemäß einem der Ansprüche 1 bis 12 gesprüht wird, zum Konditionieren und/oder Formen von Keratinfasern, insbesondere menschlichen Keratinfasern, wie dem Haar.

## Revendications

1. Dispositif aérosol contenant une composition cosmétique comprenant :
- un ou plusieurs tensioactif(s) anionique(s),
- un ou plusieurs alcool(s) gras, et
- un ou plusieurs agent(s) propulseur(s) choisis parmi les gaz comprimés,
le rapport pondéral entre la teneur totale du ou des alcool(s) gras et la teneur totale du ou des tensioactif(s) anionique(s) allant de 1,5 à 10.

2. Dispositif aérosol selon la revendication 1, **caractérisé en ce que** le ou les tensioactifs anioniques sont choisis parmi les tensioactifs anioniques carboxylates, et leurs mélanges ; de préférence parmi les acides gras en C₆-C₃₀, les acyl(C₆-C₃₀)glycinates, les acyl(C₆-C₃₀)lactylates, les acyl(C₆-C₃₀)sarcosinates, les acyl(C₆-C₃₀)glutamates, les acides alkyl-D-galactoside-uroniques, les acides alkyléthercarboxyliques, les acides alkyl(aryl en C₆-C₃₀)éthercarboxyliques, les acides alkylamidoéthercarboxyliques ; les monoesters d'alkyle en C₆-C₂₄ et d'acides polyglycoside-polycarboxyliques ; ainsi que les sels de ces composés ; et leurs mélanges ; plus préférentiellement parmi les acyl(C₆-C₂₄)sarcosinates, les acides gras en C₆-C₂₄ et leurs mélanges ; et encore plus préférentiellement parmi la stearoyl sarcosine, la myristoyl sarcosine, l'acide myristique, l'acide stéarique, l'acide palmitique, leurs sels, et leurs mélanges.

3. Dispositif selon l'une quelconque des revendications précédentes, **caractérisé en ce que** la teneur totale du ou des tensioactifs anioniques est supérieure ou égale à 0,05% en poids, de préférence supérieure ou égale 0,1% en poids, préférentiellement va de 0,1% à 5% en poids, plus préférentiellement de 0,2% à 1% en poids, par rapport au poids total de la composition.

4. Dispositif selon l'une quelconque des revendications précédentes, **caractérisé en ce que** le ou les alcools gras sont choisis parmi les alcools gras solides, et leurs mélanges, de préférence parmi les alcools gras solides saturés ou insaturés, linéaires ou ramifiés, comportant de 8 à 40 atomes de carbone, mieux parmi les alcools gras solides saturés linéaires, et leurs mélanges ; encore mieux parmi l'alcool cétylique, l'alcool stéarylique, l'alcool béhénylique, l'alcool myristique et leurs mélanges.

5. Dispositif selon l'une quelconque des revendications précédentes, **caractérisé en ce que** la teneur totale du ou des alcools gras va de 0,05% à 10% en poids, de préférence de 0,1% à 8% en poids, plus préférentiellement de 1% à 5% en poids, par rapport au poids total de la composition.

6. Dispositif selon l'une quelconque des revendications précédentes, **caractérisé en ce que** le rapport pondéral entre la teneur totale du ou des alcools gras et la teneur totale du ou des tensioactifs anioniques va de de 2 à 8, de préférence de 3 à 7, encore plus préférentiellement de 4 à 6.

7. Dispositif selon l'une quelconque des revendications précédentes, **caractérisé en ce que** le rapport pondéral entre la teneur totale en alcool(s) gras solide(s) et la teneur totale en tensioactifs anioniques carboxylates va de 1,5 à 10, de préférence de 2 à 8, plus préférentiellement de 3 à 7, encore plus préférentiellement de 4 à 6.

8. Dispositif selon l'une quelconque des revendications précédentes, **caractérisé en ce que** le ou les agents propulseurs sont choisis parmi l'air, l'azote, le gaz carbonique, et leurs mélanges, de préférence le gaz carbonique, et de préférence la teneur dudit(desdits) agent(s) propulseur(s) va de 0,5% à 10% en poids, de préférence de 1% à 5% en poids, plus préférentiellement de 1,5% à 4% en poids, par rapport au poids total de la composition.

9. Dispositif selon l'une quelconque des revendications précédentes, **caractérisé en ce que** la composition cosmétique comprend en outre un ou plusieurs corps gras différent(s) des alcools gras, de préférence un ou plus corps gras liquides différents des alcools gras, de préférence en une teneur totale allant de 0,05% à 10% en poids, préférentiellement de 0,1% à 5% en poids par rapport au poids total de la composition.

10. Dispositif selon l'une quelconque des revendications précédentes, **caractérisé en ce que** la composition cosmétique comprend en outre un ou plusieurs polymère(s) fixant(s), de préférence en une teneur totale allant de 0,05% à 10% en poids, préférentiellement de 0,1% à 5% en poids par rapport au poids total de la composition.

11. Dispositif selon l'une quelconque des revendications précédentes, **caractérisé en ce que** la composition cosmétique comprend en outre une ou plusieurs poudre(s), de préférence en une teneur totale allant de 0,05% à 10% en poids, préférentiellement de 0,1% à 5% en poids par rapport au poids total de la composition.

12. Dispositif selon l'une quelconque des revendications précédentes, **caractérisé en ce que** la composition cosmétique comprend en outre de l'eau, de préférence en une teneur allant de préférence de 10% à 99% en poids, préférentiellement de 30% à 98% en poids, mieux de 50% à 97% en poids, mieux encore de 80% à 96% en poids par rapport au poids total de la composition.

13. Procédé de traitement cosmétique des fibres kératiniques, en particulier des fibres kératiniques humaines telles que les cheveux, comprenant une étape d'application sur lesdites fibres kératiniques d'une composition cosmétique pulvérisée à partir d'un dispositif aérosol tel que défini dans l'une quelconque des revendications précédentes.

14. Utilisation d'une composition cosmétique pulvérisée à partir d'un dispositif aérosol selon l'une quelconque des revendications 1 à 12, pour le conditionnement et/ou la mise en forme des fibres kératiniques, en particulier des fibres kératiniques humaines telles que les cheveux.
